# EUROPEAN PATENT APPLICATION

(11) **EP 4 628 097 A1**
(43) Date of publication of application: **08.10.2025**
(21) Application number: 23896888.7
(22) Date of filing: 30.11.2023
(51) Int. Cl.: A61K 39/395, A61P 27/02, C07K 16/22, C07K 19/00

(54) **PREPARATION CONTAINING BISPECIFIC BINDING MOLECULE BINDING TO VEGF AND ANG2 AND USE THEREOF**

(30) Priority: 01.12.2022 CN 202211545721
(71) Applicant: Innovent Biologics (Suzhou) Co., Ltd., Suzhou, Jiangsu 215123 (CN)
(72) Inventor: LIU, Yi, Suzhou, Jiangsu 215123 (CN); HE, Bing, Suzhou, Jiangsu 215123 (CN); MA, Yidong, Suzhou, Jiangsu 215123 (CN)
(74) Representative: Sagittarius IP
(86) International application number: PCT/CN2023/135525
(87) International publication number: WO 2024/114746

(57) **Abstract**

The present invention relates to a formulation comprising a bispecific binding molecule binding to VEGF and Ang2 and uses thereof, and in particular to a pharmaceutical formulation comprising the bispecific binding molecule, a buffer, a stabilizer, and a surfactant. Furthermore, the present invention further relates to therapeutic or prophylactic use of these formulations.

## Description

### TECHNICAL FIELD

The present invention relates to the field of formulations. More specifically, the present invention relates to a formulation comprising antibodies or antigen-binding fragments thereof against vascular endothelial growth factor (VEGF/VEGF-A) and against angiopoietin-2 (ANG-2) separately, or comprising a bispecific binding molecule (e.g., an antibody) or an antigen-binding fragment thereof against both vascular endothelial growth factor (VEGF/VEGF-A) and angiopoietin-2 (ANG-2), particularly, a stable liquid formulation, and a method for preparing the antibody formulation and use of the antibody formulation.

### BACKGROUND

Angiogenesis is involved in the pathogenesis of various diseases including solid tumors, diseases associated with intraocular neovascularization, rheumatoid arthritis, and psoriasis.

VEGF is a potent and ubiquitous angiogenic factor. VEGF family members include VEGF-A, VEGF-B, VEGF-C, VEGF-D, VEGF-E, placental growth factor (PIGF), and endocrine gland-derived VEGF (EG-VEGF). Active forms of VEGF are synthesized with other VEGF family members as homodimers or heterodimers. VEGF-A exists in the following six isotypes that are generated by alternative splicing: VEGF121, VEGF145, VEGF165, VEGF183, VEGF189, and VEGF206. These isotypes differ primarily due to their bioavailability, of which VEGF165 is the major isotype. It is believed that VEGF is an important regulator for normal and disease-related angiogenesis.

In addition to the VEGF family, human angiopoietins are also thought to be involved in vascular development and postnatal angiogenesis. The human angiopoietins include a naturally occurring agonist, angiopoietin-1 (ANG-1), and a naturally occurring antagonist, angiopoietin-2 (ANG-2). The role of ANG-1 is thought to be conserved in adults, where it is widely and constitutively expressed. In contrast, ANG-2 expression is primarily restricted to sites of vascular remodeling where it is thought to block the constitutive stabilizing or maturation functions of ANG-1, allowing vessels to return to and remain in a plastic state that may be more responsive to budding signals.

In recent years, some bispecific antibodies targeting VEGF-A and ANG-2 have been developed (e.g., WO2012131078 and WO2014009465). However, the existing bispecific antibodies have poor blocking ability against VEGF and Ang2, and have too large molecular weights, resulting in lower molar concentrations at a single administration. In particular, for ocular diseases, antibodies with smaller molecular weights applied through vitreous body are typically used and require less frequent administration. Thus, there is still a need for novel bispecific binding molecules targeting VEGF-A and ANG-2, particularly suitable for ocular diseases.

There is a need for new specific antibodies that can be used to treat and/or prevent ocular diseases and formulations comprising such new antibodies, especially formulations with good stability.

### SUMMARY

The present invention meets the above needs by providing a pharmaceutical formulation comprising VHH antibodies targeting VEGF-A or ANG-2 or a bispecific binding molecule against both VEGF-A and ANG2. In particular, compared with known antibodies, the bispecific binding molecule of the present invention has a smaller molecular weight and higher molar concentration at the same mass concentration. Moreover, it has stronger VEGF A and Ang2 blocking activity, and can completely block VEGFA-induced proliferation of primary cells. Therefore, the molecule of the present invention has stronger blocking activity in clinic, can enable the molar concentration of an antibody to be higher at a single administration, maintain the efficacy of the single administration for a longer time, and reduce the frequency of ocular administration (such as intravitreal injection).

### BRIEF DESCRIPTION OF THE DRAWINGS

FIG. 1 shows the structure of bispecific binding molecules.
FIG. 2 shows that anti-VEGF A VHH antibodies can block the binding of VEGF A to VEGFR2 as determined by ELISA.
FIG. 3 shows the effect of a humanized anti-Ang2 VHH antibody on blocking the binding of Ang2 to Tie2 as determined by ELISA.
FIG. 4 shows the effect of an anti-Ang2 VHH antibody (A) and the humanized anti-Ang2 VHH antibody (B) on inhibiting hAng2-Fc-induced phosphorylation of 293-Tie2 cells as determined by ELISA.
FIG. 5 shows the effect of anti-VEGF A VHH on blocking VEGFA activation of KDR receptors as detected by an HEK293-KDR reporter method.
FIG. 6 shows the effect of the anti-VEGF A VHH antibody on inhibiting VEGF A-induced survival and proliferation of HUVEC cells as determined by CCK-8.
FIG. 7 shows the effect of a VEGF A/Ang2 bispecific binding molecule IEX04-012 on blocking VEGF activation of KDR receptors as detected by the HEK293-KDR reporter method.
FIG. 8 shows the effect of bispecific binding molecules IEX04-008, IEX04-010, and IEX04-012 on inhibiting VEGF-induced survival and proliferation of HUVEC cells.
FIG. 9 shows that the bispecific binding molecules IEX04-008, IEX04-010, and IEX04-012 block the binding of human Ang2 to Tie2 as determined by ELISA.
FIG. 10 shows that the bispecific binding molecules IEX04-008, IEX04-010, and IEX04-012 block the binding of Ang2-Fc to Tie2 as determined by flow cytometry assay.
FIG. 11 shows that the bispecific binding molecule IEX04-012 of the present invention effectively inhibits hAng2-Fc-induced phosphorylation of 293-Tie2 *in vitro* as determined by flow cytometry assay.
FIG. 12 shows that the bispecific binding molecule IEX04-012 of the present invention reduces VEGF-induced vascular endothelial cell permeability, that is, inhibits VEGF-induced HUVEC cell leakage.
FIG. 13 shows laser spot ratio statistics at grade 4 (panel A) and grade 3 or higher (panel B) in a laser-induced choroidal neovascularization model.
FIG. 14 shows retinal thickness statistics in the laser-induced choroidal neovascularization model.
FIG. 15 shows leakage area statistics in the laser-induced choroidal neovascularization model.
FIG. 16 shows H&E staining patterns of fundus tissue (A) and lesion area statistics (B) in the laser-induced choroidal neovascularization model.
FIG. 17 shows CD31 staining patterns of fundus tissue (A) and positive cell statistics (B) in the laser-induced choroidal neovascularization model.

### Definitions

Before the present invention is described in detail below, it should be understood that the present invention is not limited to the particular methodology, protocols, and reagents described herein, as these may vary. It should also be understood that the terminology used herein is only intended to describe specific embodiments rather than limit the scope of the present invention, which will be limited only by the appended claims. Unless otherwise defined, any technical and scientific term used herein has the same meaning as commonly understood by those of ordinary skill in the art to which the present invention belongs.

For the purpose of explaining this specification, the following definitions will be used, and wherever appropriate, terms used in the singular form may also include the plural form, and vice versa. It should be understood that the terms used herein are for the purpose of describing specific embodiments only, and are not intended to be limiting. The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or any two or more or all of the options.

As used herein, the term "comprise" or "include" is intended to mean that the described elements, integers or steps are included, but not to the exclusion of any other elements, integers or steps. The term "comprise" or "include" used herein, unless indicated otherwise, also encompasses the situation where the entirety consists of the described elements, integers, or steps. For example, when referring to an antibody variable region "comprising" a specific sequence, it is also intended to encompass an antibody variable region consisting of the specific sequence.

The term "VEGF" as used herein refers to an angiogenic factor. VEGF family members include VEGF-A, VEGF-B, VEGF-C, VEGF-D, VEGF-E, placental growth factor (PIGF), and endocrine gland-derived VEGF (EG-VEGF). Active forms of VEGF are synthesized with other VEGF family members as homodimers or heterodimers. VEGF-A exists in the following six isotypes that are generated by alternative splicing: VEGF121, VEGF145, VEGF165, VEGF183, VEGF189, and VEGF206. These isotypes differ primarily due to their bioavailability, of which VEGF165 is the major isotype. In some embodiments, VEGF A of the present invention refers to VEGF A from a human, e.g., VEGF 165 from a human. In one embodiment, the amino acid sequence of VEGFA of the present invention is an amino acid sequence under accession No. P15692 (uniprot database).

As used herein, the term "ANG2" refers to human angiopoietin-2 (ANG-2) (alternatively abbreviated as ANGPT2 or ANG2) which is described, for example, in Maisonpierre, P.C. et al., Science, 277 (1997) 55-60 and Cheung, A.H. et al., Genomics, 48 (1998) 389-91. Ang1 and Ang2 were discovered as ligands for tyrosine kinase family Tie selectively expressed in vascular endothelium. There are currently 4 defined members of the angiopoietin family. Angiopoietins-3 and -4 (ANG3 and ANG4) can represent a wide variety of counterparts of the same locus in mice and humans. Ang1 and Ang2 were originally identified in tissue culture experiments as an agonist and an antagonist, respectively (for ANG1, see Davis, S., et al., Cell, 87 (1996) 1161-69; for ANG2, see Maisonpierre, P.C., et al., Science 277 (1997) 55-60). All known angiopoietins mainly bind to Tie2. In some embodiments, ANG2 of the present invention refers to Ang2 from a human. In some embodiments, human Ang2 comprises an amino acid sequence under accession No. 015123 (uniprot database).

The term "multispecific binding molecule" refers to a multispecific binding molecule that is at least bispecific, such as a bispecific binding molecule, that is, the molecule comprises at least a first target-binding region and a second target-binding region, wherein the first target-binding region binds to one target or antigen, and the second target-binding region binds to another antigen or target. Accordingly, the molecule according to the present invention comprises specificities for at least two different antigens or targets. The molecule according to the present invention also encompasses a multispecific molecule comprising multiple target-binding regions/sites, such as a trispecific binding molecule. In some embodiments, the bispecific binding molecule of the present invention is a bispecific antibody.

The term "linker" as used herein refers to any molecule that enables direct connection of different portions of a bispecific binding molecule. Examples of linkers to establish covalent linkages between different portions of a molecule include peptide linkers and non-protein polymers including, but not limited to, polyethylene glycol (PEG), polypropylene glycol, polyalkylene oxide, or copolymers of polyethylene glycol and polypropylene glycol. In some embodiments, the linker is a peptide linker, which refers to an amino acid sequence that links the amino acid sequence of a first portion of a binding molecule to a second portion of the binding molecule. For example, the peptide linker may link a first target-binding region of a binding molecule to a second target-binding region. For example, the peptide linker may also link one portion of an antibody to another portion of the antibody, for example, a light chain variable region to a heavy chain variable region. Preferably, the peptide linker has a length sufficient to link two entities in a manner that maintains their conformation relative to each other without interference with the desired activities.

The peptide linker may or may not predominantly comprise the following amino acid residues: Gly, Ser, Ala or Thr. Useful linkers include glycine-serine polymers including, for example, (GS)n(SEQ ID NO:43), (GSGGS)n(SEQ ID NO:44), (GGGGS)n(SEQ ID NO:45), (GGGS)n(SEQ ID NO:46) and (GGGGS)nG(SEQ ID NO:47), wherein is an integer of at least 1 (preferably 2, 3, 4, 5, 6, 7, 8, 9 or 10). Useful linkers also include glycine-alanine polymers, alanine-serine polymers, and other flexible linkers. Preferably, the linker is (GGGGS)n(SEQ ID NO:48), wherein n = 1, 2, 3, or 4.

The term "valent" according to the present invention refers to the presence of a specified number of binding sites in a binding molecule, e.g., in an antibody molecule. Thus, the terms divalent, trivalent, and tetravalent indicate the presence of two, three, and four binding sites (target-binding regions), respectively, in a binding molecule. The bispecific binding molecule according to the present invention is at least bivalent and may be multivalent, e.g., bivalent, trivalent, tetravalent, or hexavalent.

The term "target-binding region" as used herein refers to any portion of a multispecific binding molecule, e.g., a bispecific binding molecule, that binds to a particular target or antigen. The target-binding region may be, for example, an antibody or immunoglobulin per se or an antibody fragment. Such target-binding regions may or may not have a tertiary structure independent of the remainder of BsAB, and may or may not bind to their targets as separate entities. The target-binding region may also be a receptor or a ligand, or a domain of a receptor capable of binding to a ligand.

The term "antibody fragment" includes a portion of an intact antibody. In a preferred embodiment, the antibody fragment is an antigen-binding fragment.

"Antigen-binding fragment" refers to a molecule different from an intact antibody, which comprises a portion of the intact antibody and binds to an antigen to which the intact antibody binds. Examples of the antibody fragments include, but are not limited to, Fv, Fab, Fab', Fab'-SH, F(ab')₂, a domain antibody (dAb), a linear antibody, a single-chain antibody (e.g., scFv), a single-domain antibody (e.g., VHH), a bi-valent antibody or a fragment thereof, or a camelid antibody.

"VHH", also referred to as single domain antibody (sdAb), refers to a genetically engineered antibody consisting of only the variable region of a heavy chain antibody, which contains only 3 HCDRs of the heavy chain variable region. VHH has specificity and high affinity for an antigen with only 3 HCDRs, whereas a common antibody requires 6 CDRs. The crystal structure indicates that VHH consists of 2 β-sheets forming a scaffold, which is similar to the traditional antibody VH immunoglobulin folding.

The term "target" refers to the bound substance against which the binding molecule is directed. The target may be an antigen, or may be a ligand or a receptor.

The term "antigen" refers to a molecule that induces an immune response. Such an immune response may involve antibody production or activation of specific immune cells, or both. Those skilled will understand that any macromolecules, including essentially all proteins or peptides, can be used as antigens. In addition, an antigen may be derived from recombinant or genomic DNA. As used herein, the term "epitope" refers to a moiety of an antigen (e.g., VEGF or Ang2) that specifically interacts with an antibody molecule.

"Complementarity determining region" or "CDR region" or "CDR" is a region in an antibody variable domain that is highly variable in sequence and forms a structurally defined loop ("hypervariable loop") and/or comprises antigen-contacting residues ("antigen contact sites"). CDRs are primarily responsible for binding to antigen epitopes. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2, and HCDR3, whereas the CDRs located in the light chain variable domain of the antibody are referred to as LCDR1, LCDR2, and LCDR3. In a given amino acid sequence of a light chain variable region or a heavy chain variable region, the exact amino acid sequence boundary of each CDR can be determined using any one or a combination of many well-known antibody CDR assignment systems including, e.g., Chothia based on the three-dimensional structure of antibodies and the topology of the CDR loops (Chothia *et al.*

*(*1989) Nature 342: 877-883; Al-Lazikani et al., Standard conformations for the canonical structures of immunoglobulins, Journal of Molecular Biology, 273: 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

For example, according to different CDR determination schemes, the residues of each CDR are described as follows.

| CDR | Kabat scheme | AbM scheme | Chothia scheme | Contact scheme |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 | H31-H35B | H26-H35B | H26-H32 | H30-H35B |
| (Kabat numbering system) | | | | |
| HCDR1 | H31-H35 | H26-H35 | H26-H32 | H30-H35 |
| (Chothia numbering system) | | | | |
| HCDR2 | H50-H65 | H50-H58 | H53-H55 | H47-H58 |
| HCDR3 | H95-H102 | H95-H102 | H96-H101 | H93-H101 |
| (Kabat numbering system) | | | | |

CDRs can also be determined based on having the same Kabat numbering positions as a reference CDR sequence (e.g., any of the exemplary CDRs of the present invention).

Unless otherwise stated, the term "CDR" or "CDR sequence" used herein encompasses CDR sequences determined by any one of the schemes described above.

Unless otherwise stated, residue positions of an antibody variable region (including heavy chain variable region residues and light chain variable region residues) are numbered according to the Kabat numbering system (Kabat et al., Sequences of Proteins of Immunological Interest, 5th Ed. Public Health Service, National Institutes of Health, Bethesda, Md. (1991)).

In one embodiment, the CDRs in the VHH of the present invention are determined according to the following rules: HCDR1 is determined according to AbM, and HCDR2 and HCDR3 are determined according to Kabat.

It should be noted that boundaries of CDRs in variable regions of the same antibody determined by different assignment systems may differ. That is, CDR sequences of variable regions of the same antibody defined by different assignment systems differ. Therefore, when it comes to defining an antibody with specific CDR sequences defined in the present invention, the scope of antibody also encompasses such antibodies whose variable region sequences comprise the specific CDR sequences, but having claimed CDR boundaries different from the specific CDR boundaries defined by the present invention due to a different protocol (e.g., different assignment system rules or their combinations) applied.

Antibodies with different specificities (i.e., different binding sites for different antigens) have different CDRs (under the same assignment system). However, although CDRs vary from antibody to antibody, only a limited number of amino acid positions within the CDRs are directly involved in antigen binding. The smallest overlapping region can be determined using at least two of the Kabat, Chothia, AbM, Contact, and North methods, thereby providing a "minimal binding unit" for antigen binding. The minimal binding unit may be a sub-portion of the CDR. As will be clear to those skilled in the art, residues in the remaining portions of the CDR sequences can be determined by the structure and protein folding of the antibody. Therefore, variants of any CDR presented herein are also considered. For example, in a variant of one CDR, the amino acid residue of the smallest binding unit may remain unchanged, while the remaining CDR residues defined according to Kabat or Chothia may be replaced by conservative amino acid residues.

The term "Fc region" is used herein to define the constant regions of CH2 and CH3 of immunoglobulin heavy chains, and includes Fc regions of native sequences and variant Fc regions. The native or wild-type Fc region can bind to different Fc receptors on immune cell surface, thereby causing CDC\ADCC\ADCP effector functions. Such effector functions generally require that the Fc region is associated with a binding domain (e.g., an antibody variable region). In some embodiments, the Fc region is mutated to enhance its CDC\ADCC\ADCP effector functions. In some embodiments, the Fc region is mutated to attenuate or delete its CDC\ADCC\ADCP effector functions.

"Humanized" antibody refers to an antibody comprising amino acid residues from non-human CDRs and amino acid residues from human FRs. In some embodiments, a humanized antibody will comprise at least one, or generally two of substantially all variable domains in which all or substantially all CDRs (e.g., CDRs) correspond to those of a non-human antibody, and all or substantially all FRs correspond to those of a human antibody. A humanized antibody may optionally comprise at least a portion of an antibody constant region derived from a human antibody. The "humanized form" of an antibody (such as a non-human antibody) refers to an antibody that has been humanized. "Human antibody", "fully human antibody" and "fully humanized antibody" are used interchangeably, and refer to an antibody having an amino acid sequence which corresponds to the amino acid sequence of an antibody generated by a human or human cell or derived from a non-human source that utilizes human antibody libraries or other human antibody encoding sequences. This definition of a human antibody explicitly excludes humanized antibodies comprising non-human antigen-binding residues.

As used herein, the term "anti", "binding", or "specific binding" means that the binding effect is selective for targets or antigens and may be distinguished from unwanted or non-specific interactions. The ability of a binding site to bind to a particular target or antigen may be determined by enzyme-linked immunosorbent assay (ELISA) or conventional binding assays known in the art, such as radioimmunoassay (RIA), bio-layer interferometry, MSD assay, or surface plasmon resonance (SPR).

The terms "host cell", "host cell line" and "host cell culture" are used interchangeably and refer to cells into which exogenous nucleic acids are introduced, including progenies of such cells. Host cells include "transformants" and "transformed cells", which include primary transformed cells and progenies derived therefrom, regardless of the number of passages. Progeny may not be exactly the same as parent cells in terms of nucleic acid content, and may comprise mutations. Mutant progenies having the same function or biological activities that are screened or selected from the initially transformed cells are included herein.

The term "label" used herein refers to a compound or composition which is directly or indirectly conjugated or fused to an agent, such as a polynucleotide probe or an antibody, and facilitates the detection of the agent to which it is conjugated or fused. The label itself can be detectable (e.g., a radioisotope label or a fluorescent label) or can catalyze a chemical change to a detectable substrate compound or composition in the case of enzymatic labeling. The term is intended to encompass direct labeling of a probe or an antibody by coupling (i.e., physical linking) a detectable substance to the probe or an antibody and indirect labeling of a probe or antibody by reacting with another reagent which is directly labeled. In some embodiments, the label is hFc or biotin.

The "subject" includes mammals. The mammals include, but are not limited to, domestic animals (e.g., cattle, goats, cats, dogs, and horses), primates (e.g., human and non-human primates such as monkeys), rabbits, and rodents (e.g., mice and rats). In some embodiments, the subject is a human.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate percent identity between the sequences. In one preferred embodiment, the percent identity between two amino acid sequences is determined with the Needlema and Wunsch algorithm ((1970) J. Mol. Biol., 48: 444-453; available at http://www.gcg.com) which has been integrated into the GAP program of the GCG software package, using the Blossum 62 matrix or PAM250 matrix and a gap weight of 16, 14, 12, 10, 8, 6, or 4 and a length weight of 1, 2, 3, 4, 5, or 6. In another preferred embodiment, the percent identity between two nucleotide acid sequences is determined with the GAP program of the GCG software package (available at http://www.gcg.com), using the NWSgapdna.CMP matrix and a gap weight of 40, 50, 60, 70, or 80 and a length weight of 1, 2, 3, 4, 5, or 6. A particularly preferred parameter set (and one that should be used unless otherwise stated) is a Blossum 62 scoring matrix with a gap penalty of 12, a gap extension penalty of 4, and a frameshift gap penalty of 5. The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4:11-17) which has been incorporated into the ALIGN program (version 2.0). Additionally or alternatively, the nucleic acid sequences and protein sequences described herein can be further used as "query sequences" to perform searches against public databases to, e.g., identify other family member sequences or related sequences.

The "ocular disease" described herein encompasses ocular diseases (e.g., diseases occurring in the eye) involving angiogenesis, such as ocular diseases associated with corneal neovascularization.

As used herein, "treatment" (or "treat" or "treating") refers to slowing, interrupting, arresting, alleviating, stopping, lowering, or reversing the progression or severity of an existing symptom, disorder, condition, or disease.

As used herein, "prevention" (or "prevent" or "preventing") includes the inhibition of the development or progression of symptoms of a disease or disorder, or a specific disease or disorder.

The term "vector" used herein refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of a nucleic acid to which they are operably linked. Such vectors are referred to as "expression vectors" herein.

"Subject/patient sample" refers to a collection of cells or fluids obtained from a patient or a subject. The source of tissue or cell samples can be solid tissues, e.g., from fresh, frozen and/or preserved organ or tissue samples or biopsy samples or puncture samples; blood or any blood component; body fluids such as tears, vitreous humors, cerebrospinal fluids, amniotic fluids, peritoneal fluids, or interstitial fluids; and cells from a subject at any time during pregnancy or development. In some embodiments, the tissue sample is an ocular tissue, such as vitreous body. In some embodiments, the sample is tear or vitreous humor. Tissue samples may comprise compounds which are naturally not mixed with tissues, such as preservatives, anticoagulants, buffers, fixatives, nutrients, and antibiotics.

The antibodies used in the formulation of the present invention are also referred to as antibodies of the present invention. For example, the anti-VEGF A VHH antibody in the formulation of the present invention is sometimes also referred to as the anti-VEGF A VHH antibody of the present invention.

### DETAILED DESCRIPTION

In one aspect, the present invention provides a liquid formulation comprising: (i) an anti-VEGF A VHH antibody or an anti-Ang2 VHH antibody, or a bispecific binding molecule binding to VEGF A and Ang2; (ii) a buffer, (iii) a stabilizer, and (iv) a surfactant, wherein the pH of the liquid formulation is about 5.0 to 7.5.

In some embodiments, the liquid formulation comprises an anti-VEGF A VHH antibody.

In some embodiments, the liquid formulation comprises an anti-Ang2 VHH antibody.

In some embodiments, the liquid formulation comprises a bispecific binding molecule against VEGF A and Ang2, preferably a bispecific antibody binding to VEGF A and Ang2.

In some embodiments, the anti-VEGF A VHH comprises 3 CDRs: HCDR1, HCDR2, and HCDR3, wherein
the HCDR1 comprises or consists of a sequence set forth in SEQ ID NO: 1;
the HCDR2 comprises or consists of a sequence set forth in SEQ ID NO: 2;
the HCDR3 comprises or consists of a sequence set forth in SEQ ID NO: 3;
   or
the HCDR1 comprises or consists of a sequence set forth in SEQ ID NO: 6;
the HCDR2 comprises or consists of a sequence set forth in SEQ ID NO: 7 or 10; and
the HCDR3 comprises or consists of a sequence set forth in SEQ ID NO: 8.

In some embodiments, the anti-VEGF VHH comprises or consists of an amino acid sequence set forth in SEQ ID NO: 4, 5, 9, or 11, or comprises an amino acid sequence having at least 90%, 91%, 92%, 93%, 94%, 95%, 96%, 97%, 98%, or 99% identity to the amino acid sequence set forth in SEQ ID NO: 4, 5, 9, or 11.

In some embodiments, the anti-VEGF VHH comprises an amino acid sequence having one or several (preferably no more than 10, 9, 8, 7, 6, 5, 4, 3, 2, or 1) mutations compared to an amino acid sequence set forth in SEQ ID NO: 4, 5, 9, or 11, wherein the mutations are, for example, substitutions, deletions, or additions, preferably substitutions, such as conservative substitutions.

In some preferred embodiments, the mutations are not present in the CDRs, such as HCDR1, HCDR2, or HCDR3.

In some embodiments, the anti-Ang2 VHH comprises 3 CDRs: HCDR1, HCDR2 and HCDR3, wherein
the HCDR1 comprises or consists of a sequence set forth in SEQ ID NO: 16;
the HCDR2 comprises or consists of a sequence set forth in SEQ ID NO: 17 or 20; and
the HCDR3 comprises or consists of a sequence set forth in SEQ ID NO: 18.

In some embodiments, the anti-Ang2 VHH comprises or consists of an amino acid sequence set forth in SEQ ID NO: 19 or 21, or comprises an amino acid sequence having at least 90%, 91%, 92%, 93%, 94%, 95%, 96%, 97%, 98%, or 99% identity to the amino acid sequence set forth in SEQ ID NO: 19 or 21.

In some embodiments, the anti-Ang2 VHH comprises an amino acid sequence having one or several (preferably no more than 10, 9, 8, 7, 6, 5, 4, 3, 2, or 1) mutations compared to an amino acid sequence set forth in SEQ ID NO: 19 or 21, wherein the mutations are, for example, substitutions, deletions, or additions, preferably substitutions, such as conservative substitutions.

In some preferred embodiments, the mutations are not present in the CDRs, such as HCDR1, HCDR2, or HCDR3.

In some embodiments, the bispecific binding molecule binding to VEGF A and Ang2 comprises a first target-binding region specifically binding to VEGF A and a second target-binding region specifically binding to Ang2, wherein the second target-binding region is an anti-Ang2 VHH, e.g., an anti-Ang2 VHH as described above.

In some embodiments, the first target-binding region is selected from:
a VHH specifically binding to VEGF A;
an antigen-binding fragment of an antibody, e.g., an scFv, specifically binding to VEGF A, for example, the antibody is a fully human or humanized antibody; or
a VEGF receptor (VEGF R) specifically binding to VEGF A or an extracellular domain thereof or a fusion protein comprising the extracellular domain thereof, e.g., a fusion protein of the extracellular domain with Fc.

In some embodiments, the bispecific binding molecule comprises 1, 2, 3, or 4 first or second target-binding regions. In some embodiments, the bispecific binding molecule comprises 2, 3, or 4 target-binding regions. In some embodiments, the bispecific binding molecule is bivalent, trivalent, or tetravalent. In some embodiments, the bispecific binding molecule is a bispecific antibody.

In some embodiments, the bispecific binding molecule, e.g., a bispecific antibody, has the following structure: light chain variable region VL of the anti-VEGF antibody-linker-heavy chain variable region VH of the anti-VEGF antibody-linker-anti-Ang2 VHH or
heavy chain variable region VH of the anti-VEGF antibody-linker-light chain variable region VL of the anti-VEGF antibody-linker-anti-Ang2 VHH;
wherein the anti-Ang2 VHH comprises HCDR1, HCDR2, and HCDR3, wherein the HCDR1 comprises or consists of a sequence set forth in SEQ ID NO: 16; the HCDR2 comprises or consists of a sequence set forth in SEQ ID NO: 17 or 20; and the HCDR3 comprises or consists of a sequence set forth in SEQ ID NO: 18.

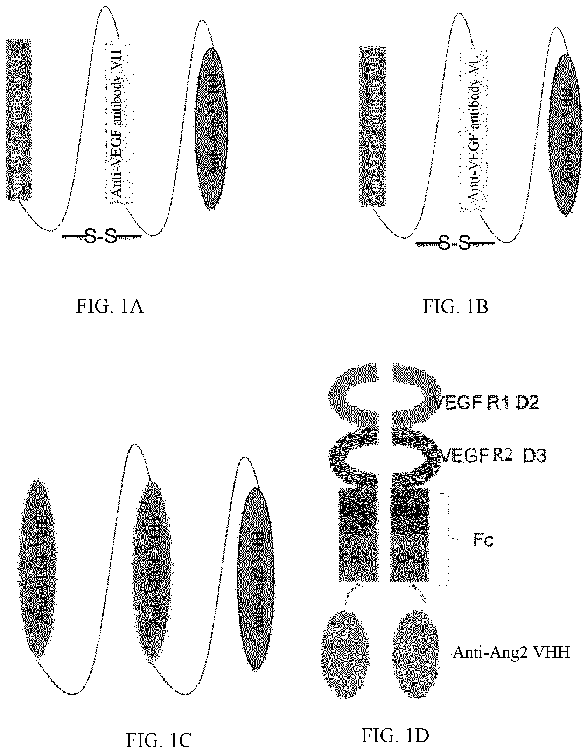

In some embodiments, the structure of the bispecific binding molecule described above is shown in FIG. 1A or FIG. 1B. In some embodiments, the bispecific binding molecule described above consists of one chain. In some embodiments, the bispecific binding molecule described above is bivalent.

In some embodiments, the light chain variable region VL of the anti-VEGF antibody comprises LCDR1, LCDR2, and LCDR3, wherein the LCDR1 comprises or consists of a sequence set forth in SEQ ID NO: 31; the LCDR2 comprises or consists of a sequence set forth in SEQ ID NO: 32; and the LCDR3 comprises or consists of a sequence set forth in SEQ ID NO: 33.

In some embodiments, the heavy chain variable region VH of the anti-VEGF antibody comprises HCDR1, HCDR2, and HCDR3, wherein the HCDR1 comprises or consists of a sequence set forth in SEQ ID NO: 35; the HCDR2 comprises or consists of a sequence set forth in SEQ ID NO: 36; and the HCDR3 comprises or consists of a sequence set forth in SEQ ID NO: 37.

In some embodiments, the heavy chain variable region VH of the anti-VEGF antibody comprises or consists of an amino acid sequence set forth in SEQ ID NO: 34, or comprises an amino acid sequence having at least 90%, 91%, 92%, 93%, 94%, 95%, 96%, 97%, 98%, or 99% identity to the amino acid sequence set forth in SEQ ID NO: 34. In some embodiments, the heavy chain variable region VH comprises an amino acid sequence having one or several (preferably no more than 10, 9, 8, 7, 6, 5, 4, 3, 2, or 1) mutations compared to an amino acid sequence set forth in SEQ ID NO: 34, wherein the mutations are, for example, substitutions, deletions, or additions, preferably substitutions, such as conservative substitutions. In some preferred embodiments, the mutations are not present in the CDRs, such as HCDR1, HCDR2, or HCDR3.

In some embodiments, the light chain variable region VL of the anti-VEGF antibody comprises or consists of an amino acid sequence set forth in SEQ ID NO: 30, or comprises an amino acid sequence having at least 90%, 91%, 92%, 93%, 94%, 95%, 96%, 97%, 98%, or 99% identity to the amino acid sequence set forth in SEQ ID NO: 30. In some embodiments, the light chain variable region VL comprises an amino acid sequence having one or several (preferably no more than 10, 9, 8, 7, 6, 5, 4, 3, 2, or 1) mutations compared to an amino acid sequence set forth in SEQ ID NO: 30, wherein the mutations are, for example, substitutions, deletions, or additions, preferably substitutions, such as conservative substitutions. In some preferred embodiments, the mutations are not present in the CDRs, such as LCDR1, LCDR2, or LCDR3.

In some embodiments, the anti-Ang2 VHH comprises or consists of an amino acid sequence set forth in SEQ ID NO: 19 or 21, or comprises an amino acid sequence having at least 90%, 91%, 92%, 93%, 94%, 95%, 96%, 97%, 98%, or 99% identity to the amino acid sequence set forth in SEQ ID NO: 19 or 21. In some embodiments, the VHH comprises an amino acid sequence having one or several (preferably no more than 10, 9, 8, 7, 6, 5, 4, 3, 2, or 1) mutations compared to an amino acid sequence set forth in SEQ ID NO: 19 or 21, wherein the mutations are, for example, substitutions, deletions, or additions, preferably substitutions, such as conservative substitutions. In some preferred embodiments, the mutations are not present in the CDRs, such as HCDR1, HCDR2, or HCDR3.

In some embodiments, the linker comprises or consists of an amino acid sequence set forth in SEQ ID NO: 23. In some embodiments, the linker between the light chain variable region and the heavy chain variable region of the anti-VEGF antibody comprises or consists of an amino acid sequence set forth in SEQ ID NO: 23, wherein, for example, n = 4. In some embodiments, the linker between the anti-VEGF variable region and the anti-Ang2 VHH comprises or consists of an amino acid sequence set forth in SEQ ID NO: 23, wherein, for example, n = 2 or 3, such as 3.

In some embodiments, the anti-VEGF A × ANG2 bispecific binding molecule comprises or consists of an amino acid sequence set forth in SEQ ID NO: 28, or comprises an amino acid sequence having at least 90%, 91%, 92%, 93%, 94%, 95%, 96%, 97%, 98%, or 99% identity to the amino acid sequence set forth in SEQ ID NO: 28. In some embodiments, the bispecific binding molecule comprises an amino acid sequence having one or several (preferably no more than 10, 9, 8, 7, 6, 5, 4, 3, 2, or 1) mutations compared to an amino acid sequence set forth in SEQ ID NO: 28, wherein the mutations are, for example, substitutions, deletions, or additions, preferably substitutions, such as conservative substitutions. In some preferred embodiments, the mutations are not present in the variable regions of the anti-VEGF antibody and the CDRs of the anti-Ang2 VHH.

In some embodiments, the bispecific binding molecule, e.g., a bispecific antibody, has the following structure:
first anti-VEGF VHH-linker-second anti-VEGF VHH-linker-anti-Ang2 VHH,
wherein the anti-Ang2 VHH comprises HCDR1, HCDR2, and HCDR3, wherein the HCDR1 comprises or consists of a sequence set forth in SEQ ID NO: 16; the HCDR2 comprises or consists of a sequence set forth in SEQ ID NO: 17 or 20; and the HCDR3 comprises or consists of a sequence set forth in SEQ ID NO: 18.

In some embodiments, the structure of the bispecific binding molecule described above is shown in FIG. 1C. In some embodiments, the bispecific binding molecule described above consists of one chain. In some embodiments, the bispecific binding molecule described above is trivalent. In some embodiments, the first anti-VEGF VHH is the same as or different from the second anti-VEGF VHH.

In some embodiments, the anti-VEGF VHH comprises HCDR1, HCDR2, and HCDR3, wherein the HCDR1 comprises or consists of a sequence set forth in SEQ ID NO: 1; the HCDR2 comprises or consists of a sequence set forth in SEQ ID NO: 2; and the HCDR3 comprises or consists of a sequence set forth in SEQ ID NO: 3;
or
the HCDR1 comprises or consists of a sequence set forth in SEQ ID NO: 6; the HCDR2 comprises or consists of a sequence set forth in SEQ ID NO: 7 or 10; and the HCDR3 comprises or consists of a sequence set forth in SEQ ID NO: 8.

In some embodiments, the anti-VEGF VHH comprises or consists of an amino acid sequence set forth in SEQ ID NO: 4, 5, 9, or 11, or comprises an amino acid sequence having at least 90%, 91%, 92%, 93%, 94%, 95%, 96%, 97%, 98%, or 99% identity to the amino acid sequence set forth in SEQ ID NO: 4, 5, 9, or 11. In some embodiments, the VHH comprises an amino acid sequence having one or several (preferably no more than 10, 9, 8, 7, 6, 5, 4, 3, 2, or 1) mutations compared to an amino acid sequence set forth in SEQ ID NO: 4, 5, 9, or 11, wherein the mutations are, for example, substitutions, deletions, or additions, preferably substitutions, such as conservative substitutions. In some preferred embodiments, the mutations are not present in the CDRs, such as HCDR1, HCDR2, or HCDR3.

In some embodiments, the anti-VEGF VHH comprises HCDR1, HCDR2, and HCDR3, wherein the HCDR1 comprises or consists of a sequence set forth in SEQ ID NO: 6; the HCDR2 comprises or consists of a sequence set forth in SEQ ID NO: 7 or 10; and the HCDR3 comprises or consists of a sequence set forth in SEQ ID NO: 8.

In some embodiments, the anti-VEGF VHH comprises or consists of an amino acid sequence set forth in SEQ ID NO: 9 or 11, or comprises an amino acid sequence having at least 90%, 91%, 92%, 93%, 94%, 95%, 96%, 97%, 98%, or 99% identity to the amino acid sequence set forth in SEQ ID NO: 9 or 11. In some embodiments, the VHH comprises an amino acid sequence having one or several (preferably no more than 10, 9, 8, 7, 6, 5, 4, 3, 2, or 1) mutations compared to an amino acid sequence set forth in SEQ ID NO: 9 or 11, wherein the mutations are, for example, substitutions, deletions, or additions, preferably substitutions, such as conservative substitutions. In some preferred embodiments, the mutations are not present in the CDRs, such as HCDR1, HCDR2, or HCDR3.

In some embodiments, the anti-Ang2 VHH comprises or consists of an amino acid sequence set forth in SEQ ID NO: 19 or 21, or comprises an amino acid sequence having at least 90%, 91%, 92%, 93%, 94%, 95%, 96%, 97%, 98%, or 99% identity to the amino acid sequence set forth in SEQ ID NO: 19 or 21. In some embodiments, the VHH comprises an amino acid sequence having one or several (preferably no more than 10, 9, 8, 7, 6, 5, 4, 3, 2, or 1) mutations compared to an amino acid sequence set forth in SEQ ID NO: 19 or 21, wherein the mutations are, for example, substitutions, deletions, or additions, preferably substitutions, such as conservative substitutions. In some preferred embodiments, the mutations are not present in the CDRs, such as HCDR1, HCDR2, or HCDR3.

In some embodiments, the linker comprises or consists of an amino acid sequence set forth in SEQ ID NO: 23. In some embodiments, the linker between the first anti-VEGF VHH and the second anti-VEGF VHH or the linker between the second anti-VEGF VHH and the anti-Ang2 VHH comprises or consists of an amino acid sequence set forth in SEQ ID NO: 23, for example, n = 2.

In some embodiments, the anti-VEGF A × ANG2 bispecific binding molecule comprises or consists of an amino acid sequence set forth in SEQ ID NO: 22, or comprises an amino acid sequence having at least 90%, 91%, 92%, 93%, 94%, 95%, 96%, 97%, 98%, or 99% identity to the amino acid sequence set forth in SEQ ID NO: 22. In some embodiments, the bispecific binding molecule comprises an amino acid sequence having one or several (preferably no more than 10, 9, 8, 7, 6, 5, 4, 3, 2, or 1) mutations compared to an amino acid sequence set forth in SEQ ID NO: 22, wherein the mutations are, for example, substitutions, deletions, or additions, preferably substitutions, such as conservative substitutions. In some preferred embodiments, the mutations are not present in the CDRs of the anti-VEGF VHH and the anti-Ang2 VHH.

In some embodiments, the bispecific binding molecule comprises one or two of the following chains:
VEGF R extracellular domain-Fc-linker-anti-Ang2 VHH,
wherein the anti-Ang2 VHH comprises HCDR1, HCDR2, and HCDR3, wherein the HCDR1 comprises or consists of a sequence set forth in SEQ ID NO: 16; the HCDR2 comprises or consists of a sequence set forth in SEQ ID NO: 17 or 20; and the HCDR3 comprises or consists of a sequence set forth in SEQ ID NO: 18.

In some embodiments, the structure of the bispecific binding molecule described above is shown in FIG. ID. In some embodiments, the bispecific binding molecule described above consists of two chains. In some embodiments, the bispecific binding molecule described above is tetravalent.

In some embodiments, the anti-Ang2 VHH comprises or consists of an amino acid sequence set forth in SEQ ID NO: 19 or 21, or comprises an amino acid sequence having at least 90%, 91%, 92%, 93%, 94%, 95%, 96%, 97%, 98%, or 99% identity to the amino acid sequence set forth in SEQ ID NO: 19 or 21. In some embodiments, the VHH comprises an amino acid sequence having one or several (preferably no more than 10, 9, 8, 7, 6, 5, 4, 3, 2, or 1) mutations compared to an amino acid sequence set forth in SEQ ID NO: 19 or 21, wherein the mutations are, for example, substitutions, deletions, or additions, preferably substitutions, such as conservative substitutions. In some preferred embodiments, the mutations are not present in the CDRs, such as HCDR1, HCDR2, or HCDR3.

In some embodiments, the VEGFR extracellular domain is an extracellular domain of VEGFR from a human. In some embodiments, the VEGFR extracellular domain comprises a second antibody-like domain of VEGFR1 (e.g., FLT1 domain 2) and a third antibody-like domain of VEGFR2 (e.g., KDR domain 3). In some embodiments, the VEGFR extracellular domain comprises a second antibody-like domain of human VEGFR1 and a third antibody-like domain of human VEGFR2. In some embodiments, the VEGFR extracellular domain comprises or consists of an amino acid sequence set forth in SEQ ID NO: 26, or comprises an amino acid sequence having at least 90%, 91%, 92%, 93%, 94%, 95%, 96%, 97%, 98%, or 99% identity to the amino acid sequence set forth in SEQ ID NO: 26. In some embodiments, the VEGFR extracellular domain comprises an amino acid sequence having one or several (preferably no more than 10, 9, 8, 7, 6, 5, 4, 3, 2, or 1) mutations compared to an amino acid sequence set forth in SEQ ID NO: 26, wherein the mutations are, for example, substitutions, deletions, or additions, preferably substitutions, such as conservative substitutions; preferably, the VEGFR extracellular domain retains a binding affinity for VEGF similar to (e.g., having at least 70%, 75%, 80%, 85%, 90%, 95%, 96%, 97%, 98%, or 99% similarity to) that of a domain set forth in SEQ ID NO: 26.

In some embodiments, the Fc is an Fc derived from human IgG1, IgG2, IgG3, or IgG4, such as a wild-type Fc, or an Fc variant known in the art. In some embodiments, the Fc comprises or consists of an amino acid sequence set forth in SEQ ID NO: 27, or comprises an amino acid sequence having at least 90%, 91%, 92%, 93%, 94%, 95%, 96%, 97%, 98%, or 99% identity to the amino acid sequence set forth in SEQ ID NO: 27.

In some embodiments, the VEGF R extracellular domain-Fc is a fusion protein of the VEGFR extracellular domain and the Fc, such as aflibercept or a derivative thereof.

In some embodiments, the VEGF R extracellular domain-Fc comprises or consists of an amino acid sequence set forth in SEQ ID NO: 25, or comprises an amino acid sequence having at least 90%, 91%, 92%, 93%, 94%, 95%, 96%, 97%, 98%, or 99% identity to the amino acid sequence set forth in SEQ ID NO: 25. In some embodiments, the VEGF R extracellular domain-Fc comprises an amino acid sequence having one or several (preferably no more than 10, 9, 8, 7, 6, 5, 4, 3, 2, or 1) mutations compared to an amino acid sequence set forth in SEQ ID NO: 25, wherein the mutations are, for example, substitutions, deletions, or additions, preferably substitutions, such as conservative substitutions; preferably, the VEGF R extracellular domain-Fc retains a binding affinity for VEGF similar to (e.g., having at least 70%, 75%, 80%, 85%, 90%, 95%, 96%, 97%, 98%, or 99% similarity to) that of a domain set forth in SEQ ID NO: 25.

In some embodiments, the linker comprises or consists of an amino acid sequence set forth in SEQ ID NO: 23, for example, n = 3.

In some embodiments, one chain of the anti-VEGF A × ANG2 bispecific binding molecule comprises or consists of an amino acid sequence set forth in SEQ ID NO: 24, or comprises an amino acid sequence having at least 90%, 91%, 92%, 93%, 94%, 95%, 96%, 97%, 98%, or 99% identity to the amino acid sequence set forth in SEQ ID NO: 24. In some embodiments, the bispecific binding molecule comprises an amino acid sequence having one or several (preferably no more than 10, 9, 8, 7, 6, 5, 4, 3, 2, or 1) mutations compared to an amino acid sequence set forth in SEQ ID NO: 24, wherein the mutations are, for example, substitutions, deletions, or additions, preferably substitutions, such as conservative substitutions. In some preferred embodiments, the mutations are not present in the CDRs of the anti-Ang2 VHH. In some embodiments, the VEGF R extracellular domain-Fc having a mutation retains a binding affinity for VEGF similar to (e.g., having at least 70%, 75%, 80%, 85%, 90%, 95%, 96%, 97%, 98%, or 99% similarity to) that of a domain set forth in SEQ ID NO: 25.

In one embodiment of the present invention, the antibody or binding molecule described herein comprises one or more amino acid mutations. In some embodiments, the amino acid mutations include amino acid substitutions, insertions, or deletions. Preferably, the amino acid change described herein is an amino acid substitution, preferably a conservative substitution.

In a preferred embodiment, the amino acid mutations described herein occur in a region outside the CDRs (e.g., in FRs). In some embodiments, the amino acid mutations described herein occur in the antibody heavy chain constant region, such as an Fc region. In a preferred embodiment, the amino acid mutations in the Fc region attenuate or delete the ADCC and/or CDC effect of the antibody.

In some embodiments, the substitution is a conservative substitution. A conservative substitution refers to the substitution of an amino acid by another amino acid of the same class, e.g., the substitution of an acidic amino acid by another acidic amino acid, the substitution of a basic amino acid by another basic amino acid, or the substitution of a neutral amino acid by another neutral amino acid.

In certain embodiments, one or more amino acid mutations may be introduced into the Fc region of the antibody provided herein, thereby producing an Fc region variant to alter one or more functional properties of the antibody, such as serum half-life, complement fixation, complement-dependent cytotoxicity, Fc receptor binding, and/or antibody-dependent cell-mediated cytotoxicity. The Fc region variant may comprise a human Fc region sequence (such as human IgG1, IgG2, IgG3, or IgG4 Fc region) comprising an amino acid mutation (such as substitution) at one or more amino acid positions.

In certain embodiments, the variable region of the antibody may need to be mutated to produce a disulfide bond, for example, to produce an scFv comprising a disulfide bond mutation.

In certain embodiments, the antibody or binding molecule provided herein can be further modified to comprise other non-proteinaceous portions known in the art and readily available. Suitable portions for derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), and glucan or poly(n-vinylpyrrolidone), polyethylene glycol, propylene glycol homopolymer, polypropylene oxide/ethylene oxide copolymer, polyoxyethylated polyol (such as glycerol), polyvinyl alcohol, and mixtures thereof.

In some embodiments, the buffer is selected from one or more of histidine, histidine salt, glutamate, phosphate, acetate, citrate, and tris(hydroxymethyl)aminomethane. The histidine salt and glutamate include, but are not limited to, salts formed by the amino acids and hydrochloric acid or sulfuric acid. The phosphate, acetate, and citrate include, but are not limited to, alkali metal salts of the corresponding acids, such as lithium, sodium or potassium salts. For example, the phosphate may be in a hydrate form or a non-hydrate form, including, but not limited to, disodium hydrogen phosphate, sodium dihydrogen phosphate, dipotassium hydrogen phosphate, potassium dihydrogen phosphate, dilithium hydrogen phosphate, lithium dihydrogen phosphate, or combinations thereof; preferably, the histidine salt is histidine hydrochloride, and the phosphate salt is a combination of disodium hydrogen phosphate and sodium dihydrogen phosphate. Preferably, the buffer is selected from a combination of histidine and histidine hydrochloride, phosphate, and histidine.

In some embodiments, the stabilizer is selected from one or more of a saccharide, a polyol, and an amino acid or a salt thereof; preferably the saccharide is selected from, but not limited to: sucrose, dextrose, lactose, maltose, trehalose, cyclodextrin, maltodextrin and glucan; the polyol is selected from, but not limited to: mannitol, sorbitol, and xylitol; the amino acid or the salt thereof is selected from one or more of the following: arginine, glycine, proline, methionine and salts thereof; the cyclodextrin is preferably hydroxypropyl-β-cyclodextrin.

In some embodiments, the stabilizer is sucrose or trehalose. In some embodiments, the stabilizer is a combination of sucrose or trehalose with an additional component selected from one or more of the following: glycine, proline, methionine, and hydroxypropyl-β-cyclodextrin.

In some embodiments, the surfactant is a non-ionic surfactant, including, but not limited to, alkyl poly(ethylene oxide); polysorbates, such as polysorbate-20, polysorbate-80, polysorbate-60 or polysorbate-40; Pluronic; and the like. In some preferred embodiment, the liquid formulation of the present invention comprises polysorbate-80 as the surfactant.

In some embodiments, the concentration of the antibody or bispecific binding molecule in the liquid formulation of the present invention is about 1-200 mg/mL, for example, about 1-150 mg/mL, about 10-190 mg/mL, about 20-180 mg/mL, about 30-170 mg/mL, about 30-150 mg/mL, about 30-170 mg/mL, about 30-100 mg/mL, about 30-100 mg/mL, e.g., about 5, about 10, about 15, about 20, about 25, about 30, about 40, about 50, about 60, about 70, about 80, about 90, about 100, about 110, about 120, about 130, about 140, about 150, about 160, about 170, about 180, about 190 or about 200 mg/mL, preferably about 10-160 mg/mL, more preferably about 30-150 mg/mL.

In some embodiments, the concentration of the buffer in the liquid formulation of the present invention is about 0.5-200 mM, about 1-100 mM, about 5-50 mM, about 5-30 mM, about 5-20 mM, about 5-15 mM, about 8-12 mM, e.g., about 5, about 10, about 15, about 20, about 25, about 30, about 40, about 50, about 60, about 70, about 80, about 90, about 100, about 110, about 120, about 130, about 140, about 150, about 160, about 170, about 180, about 190 or about 200 mM, preferably about 5-50 mM, more preferably about 5-30 mM. When the buffer contains a plurality of components, the concentration of each component may be selected from the following ranges or values: 0.2-100 mM, about 0.5-50 mM, about 2-25 mM, about 2-15 mM, about 1-10 mM, about 2-8 mM, about 1-3 mM, 0.5-4 mM, about 7-9 mM, about 6-10 mM, about 5-10 mM, e.g., about 1, about 2, about 3, about 4, about 5, about 6, about 7, about 8, about 9, about 10, about 15, about 20, about 25, about 30, about 40, about 50, about 60, about 70, about 80, about 90 or about 100 mM.

In some embodiments, the concentration of the stabilizer in the liquid formulation of the present invention is about 1-1000 mM, about 10-1000 mM, about 20-800 mM, about 30-700 mM, about 50-800 mM, about 50-500 mM, about 100-400 mM, about 100-300 mM, about 200-350 mM, about 200-300 mM, about 250-280 mM, about 200-400 mM or about 100-200 mM, e.g., about 10, about 20, about 50, about 80, about 100, about 150, about 200, about 300, about 400, about 500, about 600, about 700, about 800, about 900 or about 1000 mM, preferably about 50-800 mM, more preferably about 50-500 mM. When the stabilizer contains a plurality of components, the concentration of each component may be selected from the following ranges or values: about 20-800 mM, about 30-700 mM, about 50-800 mM, about 50-500 mM, about 100-400 mM, about 100-300 mM, about 200-350 mM, about 200-300 mM, about 250-280 mM, about 200-400 mM or about 100-200 mM, about 0.5-100 mM, about 1-50 mM, about 2-50 mM, about 5-20 mM, about 8-12 mM, e.g., about 1, about 2, about 3, about 4, about 5, about 10, about 20, about 30, about 50, about 80, about 100, about 150, about 200, about 300, about 400, about 500 or about 600 mM.

In some embodiments, the concentration of the surfactant in the liquid formulation of the present invention is from about 0.01-10 mg/mL, e.g., about 0.05-5, about 0.05-2, about 0.1-1, about 0.1-5, about 0.2-2, about 0.3-1, about 0.2-0.4 mg/mL, about 0.4-0.8 or about 0.5-0.6 mg/mL, e.g., about 0.2, about 0.3, about 0.4, about 0.5, about 0.6, about 0.7 or about 0.8 mg/mL, preferably from about 0.05-5 mg/mL, more preferably from 0.05-2 mg/mL.

In some embodiments, the pH of liquid formulations of the present invention is about 5.5-7.2, 6.0-7.0, 6.1-7.0, 6.2-7.0, 6.2-6.6 or 6.3-6.8, e.g., about 5.0, about 5.2, about 5.4, about 5.6, about 5.8, about 6.0, about 6.1, about 6.2, about 6.3, about 6.4, about 6.5, about 6.6, about 6.7, about 6.8, about 6.9, about 7.0, about 7.1 or about 7.2, preferably about 6.1-7.0, more preferably about 6.3-6.8.

In some embodiments, the liquid formulation of the present invention has an osmolality of 200-400 mOsmol/kg or 250-350 mOsmol/kg, e.g., an osmolality of 350 mOsmol/kg.

In some embodiments, the liquid formulation is a pharmaceutical formulation, preferably an injection, more preferably an intravitreal injection, or an eye drop.

In some embodiments, the liquid formulation of the present invention comprises:
(i) the bispecific binding molecule at about 10-160 mg/mL, preferably 30-150 mg/mL;
(ii) the buffer at about 5-20 mM, the buffer being histidine or a combination of histidine and histidine hydrochloride;
(iii) trehalose at about 200-350 mM; and
(iv) polysorbate 80 at about 0.1-1 mg/mL; wherein
a pH of the liquid formulation is about 6.1-7.0, preferably about 6.5.

In some embodiments, the liquid formulation of the present invention comprises:
(i) the bispecific binding molecule at about 10-160 mg/mL, preferably 30-150 mg/mL;
(ii) histidine at about 8-12 mM;
(iii) trehalose at about 200-300 mM; and
(iv) polysorbate 80 at about 0.2-0.4 mg/mL; wherein
the pH of the liquid formulation is about 6.1-6.6, preferably about 6.5.

In some embodiments, the liquid formulation of the present invention comprises:
(i) the bispecific binding molecule at about 10-160 mg/mL, preferably 30-150 mg/mL;
(ii) histidine at about 1-3 mM and histidine hydrochloride at about 7-9 mM;
(iii) trehalose at about 250-280 mM; and
(iv) polysorbate 80 at about 0.2-0.4 mg/mL; wherein
the pH of the liquid formulation is about 6.1-6.6, preferably about 6.5.

In some embodiments, the liquid formulation of the present invention is the formulation disclosed in Examples 15-17. In addition, based on the formulations disclosed in Examples 15-17, embodiments resulting from a variation of 50%, 40%, 30%, 20%, 10% or 5% in the concentrations of the components in such formulations, and a variation of 5%, 4%, 3%, 2%, 1% or 0.5% in the pH values, are also included in the present application.

The liquid antibody formulation of the present invention may or may not comprise other excipients. For example, the antibody liquid formulation of the present invention may further comprise a tonicity modifier. The tonicity modifier may be selected from the group consisting of sodium acetate, sodium lactate, sodium chloride, potassium chloride and calcium chloride.

These and other known pharmaceutical excipients and/or additives suitable for use in the formulation of the present invention are well known in the art, for example, as listed in *"*The Handbook of Pharmaceutical Excipients, 4th edition, edited by Rowe et al., American Pharmaceuticals Association (2003); and Remington: the Science and Practice of Pharmacy, 21st edition, edited by Gennaro, Lippincott Williams & Wilkins (2005)".

It should be appreciated that, unless otherwise specified, the liquid formulation of the present invention further comprises a vehicle including, but not limited to, for example, purified water such as ultrapure water, water for injection, sterile water, double distilled water, and the like. The liquid formulation of the present invention may be prepared or formulated using pharmaceutically acceptable solvents or solutions known in the art. Such pharmaceutically acceptable solvents or solutions include, but are not limited to, for example, purified water such as ultrapure water, water for injection, sterile water, double distilled water, normal saline, Ringer's solution, glucose injection, and the like.

In some embodiments, the anti-Ang2 VHH antibody can specifically bind to Ang2, such as human Ang2, e.g., with high affinity.

In some embodiments, the anti-VEGF A VHH antibody can specifically bind to VEGF A, such as human VEGF A, e.g., with high affinity.

In some embodiments, the bispecific binding molecule can specifically bind to Ang2 and VEGFA, such as human Ang2 and human VEGFA, e.g., with high affinity.

In some embodiments, the anti-Ang2 antibody or the anti-VEGFA antibody or the bispecific binding molecule has one or more of the following properties:
(i) the anti-Ang2 antibody or bispecific binding molecule has an inhibitory effect on Ang2-induced Tie2 phosphorylation;
(ii) the anti-Ang2 antibody or bispecific binding molecule has a blocking effect on the binding between Ang2 and Tie2;
(iii) the anti-VEGFA antibody or bispecific binding molecule has a blocking effect on VEGFA activation-related receptor signaling pathways, e.g., as detected by KDR reporters;
(iv) the anti-VEGFA antibody or bispecific binding molecule has a blocking effect on the binding between VEGFA and VEGFR;
(v) the anti-VEGFA antibody or bispecific binding molecule has an inhibitory effect on VEGF A-induced survival and proliferation of cells (e.g., primary cells, e.g., vascular endothelial cells, e.g., human umbilical vein endothelial cells, e.g., HUVECs);
(vi) the bispecific binding molecule has an inhibitory effect on the leakage of vascular endothelial cells (e.g., human umbilical vein endothelial cells, e.g., HUVECs); and
(vii) the bispecific binding molecule has an inhibitory effect on neovascularization *in vivo* or *in vitro,* e.g., inhibiting fundus or retinochoroidal neovascularization, e.g., inhibiting the leakage caused by the neovascularization, e.g., protecting vascular integrity.

In another aspect, the present invention provides a solid formulation obtained by solidifying the liquid formulation of the present invention. The solidification treatment is implemented by, e.g., crystallization, spray drying, or freeze drying. In one preferred embodiment, the solid formulation is, e.g., in the form of a lyophilized powder for injection. The solid formulation can be reconstituted in a suitable vehicle prior to use to give a reconstituted formulation of the present invention. The reconstituted formulation is also a liquid formulation of the present invention. In one embodiment, the suitable vehicle is selected from purified water such as ultrapure water, water for injection, organic solvents for injection (including but not limited to, oil for injection, ethanol, propylene glycol, and the like), and combinations thereof.

The formulation of the present invention (including the solid formulation and the liquid formulation) can be stably stored for a long period of time, e.g., at least 24 months or longer. In one embodiment, the formulation of the present invention can be stably stored at about -80 °C to about 45 °C, e.g., -80 °C, about -30 °C, about -20 °C, about 0 °C, about 5 °C, about 25 °C, about 35 °C, about 38 °C, about 40 °C, about 42 °C or about 45 °C for at least 10 days, at least 20 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer.

In one embodiment, the stability of the liquid formulation of the present invention is visually inspected after storage, wherein the liquid formulation of the present invention remains a clear to slightly opalescent, colorless to pale yellow liquid free of particles in appearance. In one embodiment, no visible particles exist in the formulation upon visual inspection under a clarity detector. In one embodiment, the stability of the liquid formulation of the present invention is tested after storage by determining the change in protein content, wherein the change rate in protein content is no more than 20%, preferably no more than 10%, e.g., 7-8%, and more preferably no more than 5%, relative to an initial value on day 0 of storage, as measured, for example, by the ultraviolet spectrophotometry (UV) method. In one embodiment, the stability of the liquid formulation of the present invention is tested after storage by determining the change in purity of the liquid formulation of the present invention, wherein the change in monomer purity is no more than 10%, e.g., no more than 5%, 4% or 3%, e.g., 1-2%, preferably no more than 1%, relative to an initial value on day 0 of storage, as measured by size exclusion-high performance liquid chromatography (SEC-HPLC). In one embodiment, the stability of the liquid formulation of the present invention is tested after storage by determining the change in purity of the formulation of the present invention, wherein the change in monomer purity is reduced by no more than 10%, e.g., no more than 5%, 4%, 3%, 2%, or 1%, as measured by non-reduced and/or reduced sodium dodecyl sulphate capillary electrophoresis (CE-SDS). In one embodiment, the stability of the liquid formulation of the present invention is tested after storage by imaged capillary isoelectric focusing (iCIEF), wherein the total change in charge variants (main species, acidic species and basic species) of the antibody is no more than 30%, e.g., no more than 20%, no more than 10%, no more than 5%, no more than 2%, or no more than 1%, relative to an initial value on day 0 of storage.

In another aspect, the present invention provides a delivery device comprising the liquid formulation or the solid formulation of the present invention. In one embodiment, the delivery device of the present invention is provided in the form of a pre-filled syringe comprising the liquid formulation or the solid formulation of the present invention, e.g., for use in intravitreal, intravenous, subcutaneous, intradermal or intramuscular injection, or intravenous infusion.

In another aspect, the present invention provides a method for preparing the liquid formulation of the present invention, comprising the following steps:
i, providing an isolated and purified antibody or bispecific binding molecule of the present invention, and optionally adding to an ultrafiltration centrifuge tube, and concentrating by centrifugation;
ii, preparing a solution of a buffer and a stabilizer, preferably an aqueous solution, wherein preferably, the identity and concentration of the buffer and the stabilizer, and pH of the solution are as defined above in the liquid formulation of the present invention,
iii, ultrafiltering the antibody of step i into the solution of step ii,
iv, adjusting the resultant protein solution to a concentration defined for the liquid formulation of the present invention;
v, adding a surfactant or a solution thereof, preferably an aqueous solution, and adjusting the final concentration of the surfactant to a concentration defined for the liquid formulation of the present invention;
vi, optionally, aseptically filtering the solution of step v; and
vii, optionally, aliquoting the solution into vials and sealing the vials with rubber stoppers and aluminum-plastic caps to give the product.

In another aspect, the present invention provides a method for preventing or treating an ocular disease in a subject, comprising administering to the subject the formulation of the present invention.

In some embodiments, the patient has VEGF, e.g., VEGF A, and/or Ang2 (e.g., at an elevated level, e.g., at a nucleic acid or protein level).

In some embodiments, the ocular disease includes, but is not limited to, ocular diseases associated with angiogenesis, such as ocular diseases associated with corneal neovascularization.

In some embodiments, the ocular disease treatment will benefit from inhibition of VEGF, e.g., VEGF A, and/or Ang2, at a nucleic acid or protein level.

In other aspects, the present invention provides use of the formulation of the present invention in manufacturing or preparing a medicament or a delivery device, wherein the medicament is for use as described herein, for example for preventing or treating a related disease or condition mentioned herein.

In some embodiments, the formulation of the present invention delays the onset of the condition and/or symptoms associated with the condition.

In some embodiments, the formulation of the present invention can also be administered in combination with one or more other therapies, e.g., therapeutic modalities and/or other therapeutic agents, for the use described herein, e.g., for use in preventing or treating a related disease or condition mentioned herein.

The route of administration of the formulations of the present invention is in accordance with known methods, e.g., topical administration, e.g., intraocular administration or conjunctival administration. In some embodiments, the administration is performed by injection or instillation.

These and other aspects and embodiments of the present invention are illustrated in the drawings (brief description of the drawings follows) and in the following detailed description of the present invention and are described in the following examples. Any or all of the features described above and throughout the present invention may be combined in various embodiments of the present invention. The following examples further illustrate the present invention. However, it should be understood that the examples are described by way of illustration rather than limitation, and various modifications may be made by those skilled in the art.

### Example 1. Preparation of Phage Immune Library

### Construction of alpaca immune or synthetic library

1.1 Two healthy adult alpacas (Chengdu NBbiolab, Co. Ltd.) were selected, 0.5 mg of recombinant protein antigen VEGFA or Ang2 (Beijing Sino Biological) and Freund's adjuvant were mixed homogeneously according to a ratio of 1:1, and the alpacas were immunized by subcutaneous injection at multiple sites on the back for four times with an immune interval of 2 weeks.
1.2 Alpaca peripheral blood (50 mL) was collected, lymphocytes were isolated, 1 mL of Trizol reagent was added per 2.5 × 10⁷ viable cells, and the total RNA was extracted using a chloroform/isopropanol precipitation method. Reverse transcription was performed using PrimeScript reverse transcription kit (Takara) by taking 10 µg of RNA as a template. The first round of PCR reaction was performed by taking cDNA as a template and using a forward primer Alp-VhL and a reverse primer Alp-2b/2cR to obtain products from the first round of PCR. The second round of PCR reaction was performed by taking the product from the first round of PCR as a template and using a forward primer Alp-VhF and a reverse primer Alp-JHR-SalI to obtain products from the second round of PCR. The pC3-HF vector and the product from the second round of PCR were subjected to double digestion using SacI and SalI (Thermo), respectively, the digested products were reacted with T4 ligase (Thermo), and TG1 competent cells were electrotransformed to construct a VHH antibody library. The bacterial liquid was cryopreserved at -80 °C.

The thawed bacterial liquid was inoculated into 100 mL of YT-AG medium (Shanghai Sangon Biotech), an M13KO7 helper phage was added for infection, and the bacterial cell pellet was resuspended with 2×YT-AK medium (Shanghai Sangon Biotech) and cultured at 37 °C and at 200 rpm overnight. The culture supernatant was collected, and a recombinant phage was prepared using a PEG/NaCl precipitation method.
1.3 The recombinant phage was subjected to 3 rounds of panning experiments using the biotin-labeled antigen VEGFA (ACRO) or Ang2 (Beijing Sino Biological). 50 µL of M280 magnetic beads (Thermo) and an appropriate amount of biotin-labeled antigens were added to each tube, and the mixture was incubated at room temperature for 30 min. 1 × 10¹² cfu of recombinant phage was added, and the mixture was incubated at room temperature for 1 h. The resulting mixture was washed 10 times with 1 mL of PBST, each time for 5 min. Finally, 0.5 mL of glycine buffer with pH of 2.5 was added, the antigen-bound recombinant phage was eluted, TG1 was infected, and the cells were cultured overnight. The recombinant phage was prepared for the next round of panning experiment, and TG1 bacterial clones with positive VHHs were identified.
1.4 The binding activity was detected by Binding ELISA and the clones were sequenced.

VEGF A and Ang2 antigens (Beijing Sino Biological) were taken in advance and diluted to 0.5 µg/mL with PBS buffer to coat a 96-well ELISA plate, and the plate was stored in a refrigerator at 4 °C overnight. The antigen-coated plate was washed 3 times with PBST, a blocking reagent was added to 300 µL/well, and the plate was left to stand at room temperature for 1 h. The plate was washed 3 times with PBST, 80 µL of blocking reagent and 20 µL of expression supernatant of the TG1 strain with positive VHHs identified in step 1.3 described above were added, and the plate was shaken at room temperature for 1 h.

The plate was washed 3 times with PBST, 100 µL of Anti-Flag/HRP secondary antibody (Sigma) diluted with the blocking reagent was added per well, and the plate was shaken at room temperature for 40 min. The plate was washed 6 times with PBST, TMB chromogenic solution was added to 100 µL/well, and the color development was performed in the dark for 5-15 min. Then 100 µL/well of stop solution was added. The plate was read on a microplate reader, the OD450 nm absorbance values were measured, and bacterial clones with a reading value more than 0.5 were selected and sent to Genewiz for sequencing. A single clone of TG1 strain containing each corresponding VHH sequence was selected, and glycerol was added. The mixture was cryopreserved in a refrigerator at -80 °C.

### Example 2. Production, Purification and Humanization of Prokaryotic Antibody

According to the present invention, antibody sequences in anti-VEGFA or Ang2 positive phages were obtained by using molecular biology techniques, and the obtained TG1 single clones containing positive VHHs were used to be expressed and purified to obtain VHH antibody proteins.

The TG1 strain containing VHH expression plasmids identified in Example 1 was inoculated into 800 mL of LB-Amp medium and cultured at 37 °C and at 200 rpm until the OD600 value was 0.5-0.6. 1 mM IPTG was added into the bacterial liquid to induce expression, and the strain was cultured overnight at 28 °C and at 200 rpm. The culture supernatant was collected. After centrifugation, 15 mL of PB and 1 mg/mL of polymyxin were added to resuspend the bacteria, and the mixed solution was centrifuged again and filtered through a 0.22 µm filter membrane. The bacterial lysate was passed through a 1 mL Ni Sepharose pre-column, and the column was washed 2 times with PBS. The target protein was eluted with 0.5 M imidazole, and the protein concentration was measured by an ultraviolet method. The protein concentration of the eluted target protein was measured by the ultraviolet method, and the eluted target protein was aliquoted into multiple tubes and stored in a refrigerator at -40 °C. The obtained antibody solution was subsequently referred to as a supernatant.

The amino acid sequences of CDRs and VHHs of 2 anti-VEGF A VHH antibodies (LA42F8 and LA46E11) and 1 anti-Ang2 VHH antibody (LA24C11) obtained in the present invention, as well as sequence numbers are shown in the sequence listing.

Then, the immune library antibodies LA42F8, LA46E11, and LA24C11 obtained above were humanized. The steps are as follows:
(1) determining a CDR loop structure;
(2) searching a human germline sequence database for the closest homologous sequences for each V/J region of the heavy chain;
(3) screening the human germlines for the highest match in heavy and light chains and a minimum quantity of back mutations;
(4) constructing the CDRs of the chimeric antibody onto the framework of a human antibody;
(5) determining the positions of amino acids that maintained the CDR functions in the framework based on the sequences and structural features;
(6) adding back mutations (back to the input amino acids) at important positions identified; and
(7) optimizing amino acids at risk sites.

The amino acid sequences of CDRs and heavy chain variable regions of 3 humanized antibodies obtained in the present invention, namely humanized VHH antibodies LA42F8.5, LA46E11.8, and LA24C11.10 are shown in the attached sequence listing.

The expression and preparation of LA42F8, LA46E11, LA24C11, and humanized antibodies LA42F8.5, LA46E11.8, and LA24C11.10 described above in eukaryotic cells were as follows:
The humanized antibody sequences obtained above were cloned into pcDNA3.1 (Invitrogen), and plasmids containing the antibody sequences were obtained, respectively.

Expi-293 cells (Invitrogen) were passaged according to a desired transfection volume. The cell density was adjusted to 1.5 × 10⁶ cells/mL the day before transfection. The cell density on the day of transfection was approximately 3 × 10⁶ cells/mL. 1/10 of the final volume of F17 medium (Gibco, A13835-01) was taken as a transfection buffer. An appropriate amount of plasmids was added, and the mixture was mixed homogeneously. An appropriate amount of polyethylenimine (PEI) (Polysciences, 23966) was added to the plasmids (the ratio of plasmids to PEI was 1:3 in the 293F cells), mixed and incubated at room temperature for 10 min, resulting in a DNA/PEI mixture. After being resuspended with the DNA/PEI mixture, the cells were introduced at 36.5 °C, 8% CO₂. After 24 h, the cells were supplemented with FEED (Sigma) with 2% of the transfection volume, and were incubated at 36.5 °C, 8% CO₂ at 120 rpm. On Day 6 days of subculture or until a viability fell below 60%, the cell supernatant was collected and purified.

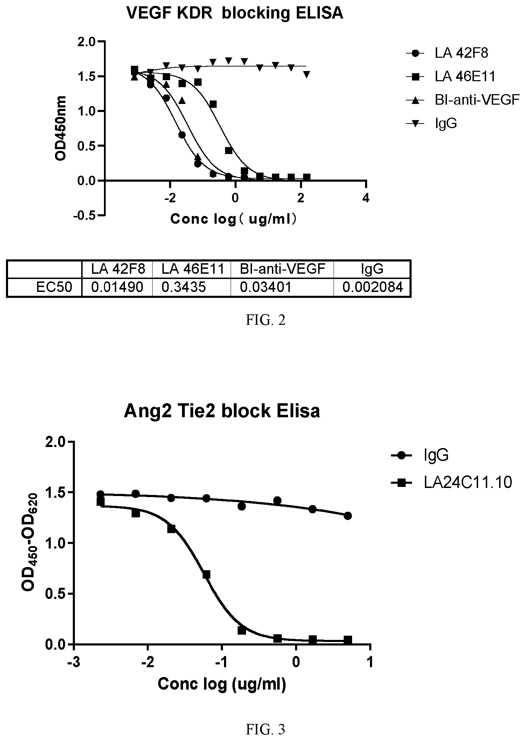

The gravity column for purification was treated with 0.5 M NaOH overnight. Glass containers were washed with distilled water and dried at 180 °C for 4 h, and a purification column was obtained. Before purification, the cell supernatant collected above was centrifuged at 4500 rpm for 30 min, and the cells were discarded. The supernatant was filtered through a 0.22 µL filter. Protein A column (Hitrap Mabselect Sure 5 × 5 mL, GE, 11-0034-95) was equilibrated with 10 mL of binding buffer (20 mM sodium phosphate, 150 mM NaCl, pH 7.0). The filtered supernatant was loaded to the purification column, which was then re-equilibrated with 15 mL of binding buffer. 5 mL of eluent buffer (citric acid + sodium citrate 0.1 M, pH 3.5) was added. The eluate was collected, and 80 µL of Tris-HCl was added per mL of eluate. The buffer of the collected antibodies was changed into PBS (Gibco, 70011-044) by ultrafiltration/diafiltration, and the concentrations were measured. Except for the antibodies of the present invention used for detection in Table 3, FIG. 2, FIG. 4A, and FIG. 5A, the antibodies used in the examples were all expressed and purified using these methods unless the supernatant was specifically mentioned subsequently. Similarly, the coding nucleic acids of negative control IgG, positive control BI-anti-VEGF, BI836880, and Faricimab (sequences are shown in sequence listing) were cloned into pcDNA3.1, transfected into Expi-293 cells, expressed, and purified, and the methods were same as those in Example 2.

### Example 3. Binding Kinetics of Chimeric Antibodies of the Present Invention for Antigens as Determined by Bio-Layer Interferometry

The equilibrium dissociation constant (KD) for binding of the antibodies of the present invention to human Ang2 was determined by bio-layer interferometry (ForteBio). A ForteBio affinity assay of prior art was performed (Estep, P., et al., High throughput solution Based measurement of antibody-antigen affinity and epitope binning, MAbs, 2013.5(2): 270-8).

Half an hour before the experiment, an appropriate number of AMQ (Pall, 1506091) (for sample detection) or AHQ (Pall, 1502051) (for positive control detection) sensors depending on the number of samples were soaked in SD buffer (PBS 1×, BSA 0.1%, Tween-20 0.05%).

100 µL of SD buffer, VHH antibodies prepared above in Example 2, and antigens [including human Ang2 (Beijing Sino Biological) and human VEGF165 (R&D)] were added to a 96-well black polystyrene half-area microplate (Greiner, 675076), respectively. The sensors were arranged according to the positions of the samples. The instrument settings were as follows: the operation procedures were Baseline, Loading -1 nm, Baseline, Association, and Dissociation; the run time of each procedure was dependent on the rates of association and dissociation; the rotation speed was 400 rpm, and the temperature was 30 °C. The K_{D} values were analyzed by ForteBio analysis software. In the experiments performed by the assays described above, the affinities of the antibodies are shown in Table 1:

**Table 1. Affinity constants (equilibrium dissociation constants) for monovalent bindings of antigens and antibodies by ForteBio assay**

| Antibody | Antigen | K_{D} (M) | Kₒₙ (1/Ms) | k_{dis} (1/s) |
|---|---|---|---|---|
| LA42F8.5 | VEGF 165 | ND | ND | ND |
| LA42F8 | | 6.11E-09 | 1.89E+05 | 1.16E-03 |
| LA46E11.8 | | ND | ND | ND |
| LA46E11 | | 7.741E-09 | 1.84E+05 | 1.42E-03 |
| LA24C11 | Ang2 | 2.18E-09 | 7.61E+05 | 1.66E-03 |
| LA24C11.10 | | ND | ND | ND |

| | | | | |
|---|---|---|---|---|
| ND denotes not detected | | | | |

**Table 2. Affinity constants (equilibrium dissociation constants) for bivalent bindings of antigens and antibodies by ForteBio assay**

| Antibody | Antigen | K_{D} (M) | Kₒₙ(1/Ms) | k_{dis}(1/s) |
|---|---|---|---|---|
| LA42F8.5 | VEGF 165 | 2.00E-10 | 1.06E+06 | 2.13E-04 |
| LA42F8 | | 8.43E-10 | 2.37E+05 | 2.00E-04* |
| LA46E11.8 | | 7.88E-10 | 4.21E+05 | 3.32E-04 |
| LA46E11 | | 1.11E-09 | 1.79E+05 | 2.00E-04* |
| LA24C11 | Ang2 | 8.67E-10 | 2.31E+05 | 2.00E-04* |
| LA24C11.10 | | 8.28E-10 | 2.42E+05 | 2.00E-04* |

| | | | | |
|---|---|---|---|---|
| * denotes dissociation constants exceeding ForteBio detection limits | | | | |

### Example 4. ELISA Blocking Assay of Anti-VEGF A VHH Antibodies

This example demonstrates the blocking effect of the anti-VEGF A VHH of the present invention on the binding of hVEGF A to a receptor KDR. SA (Thermo, Catalog No. 21125) was diluted to 1 µg/mL, and 100 µL/well of SA was plated in a microplate at 4 °C overnight. The plate was washed 3 times with PBST and blocked with 3% BSA for 1.5 h. The plate was washed 3 times with PBST, 50 ng/mL of biotin-labeled VEGF A165 (ACRO, Catalog No. VE5-H8210) was added, and the plate was incubated for 1.5 h. The LA42F8 supernatant, LA46E11 supernatant, negative control IgG, and positive control BI-anti-VEGF (50 µL) of the antibodies (with an initial concentration of 150 µg/mL, 3-fold serial dilution) prepared in Example 2 were incubated with VEGFR-Fc (Beijing Sino Biological, Catalog No. 10012-H02H, with a final concentration of 0.2 µg/mL) for 20 min in advance, and then the mixtures were added to the plate. The plate was washed 3 times with PBST, an anti-human Fc HRP antibody (Bethyl, Catalog No. A80-104P) (1:10000) was added, and then the plate was incubated for 30 min. The plate was washed 6 times with PBST, the color development was performed with TMB for 5 min, and OD450 nm was read after stopping.

The blocking results of 3 anti-VEGF VHH antibodies obtained in the present invention are shown in FIG. 2. FIG. 2 shows that the candidate molecules LA42F8 and LA46E11 antibodies can completely block the binding of VEGF A to VEGFR2.

### Example 5. ELISA Blocking Assay of Anti-Ang2 VHH Antibody

This example demonstrates the blocking effect of the anti-Ang2 VHH antibody of the present invention on the binding of hAng2-biotin (R&D, Catalog No. BT623B/CF) to a Tie2 protein.

Tie2-Fc (Beijing Sino Biological, Catalog No. 10700-H03H) was diluted to 2 µg/mL, and 100 µL/well of Tie2-Fc was plated in a microplate at 4 °C overnight. The plate was washed 3 times with PBST and blocked with 3% BSA for 1.5 h. The plate was washed 3 times with PBST, each of the purified LA24C11.10 and negative control IgG (50 µL) prepared in Example 2 was incubated with hAng2-biotin (with a final concentration of 0.2 µg/mL) for 20 min in advance, and then the mixtures were added to the plate. The plate was washed 3 times with PBST, Avidin HRP (1:2000) was added, and the plate was incubated for 35 min. The plate was washed 6 times with PBST, the color development was performed with TMB for 5 min, and OD450 nm was read after stopping.

The blocking results of the anti-Ang2 VHH antibody obtained in the present invention are shown in Table 3, and the candidate molecule LA24C11 has a blocking effect on the binding of Ang2 to a receptor Tie2.

**Table 3. Single-point blocking ELISA of purified antibody**

| | | Ang2-Bio (Sino) | | Ang2-Bio (R&D, Catalog No. BT623B/CF) | | Ang2-Bio (R&D, Catalog No. BT623B/CF) | |
|---|---|---|---|---|---|---|---|
| Clone No. | Initial concentration of antibody¹ (mg/mL) | Antibody' diluted in a 1:10 ratio (OD450nm) | Antibody' diluted in a '1:100 ratio (OD450nm) | Antibody' diluted in a 1:10 ratio (OD450nm) | Antibody' diluted in a '1:100 ratio (OD450nm) | Supernatant ² (OD450nm) | Blank (without antibody) (OD450nm) |
| 24C11 | 0.556 | 0.09 | 0.20 | 0.07 | 0.07 | 0.12 | 3.45 |

| | | | | | | | |
|---|---|---|---|---|---|---|---|
| ¹ The antibody refers to the purified prokaryotic supernatant prepared in Example 2, with an initial concentration of 0.556 mg/mL, followed by 1:10 or 1:100 dilution; ² The supernatant refers to a prokaryotic supernatant without purification. | | | | | | | |

Similar to Example 4 (using Ang2-Bio (R&D, Catalog No. BT623B/CF)), the blocking effect of LA24C11.10 on the binding of Ang2 to Tie2 was detected by ELISA blocking experiments. The results are shown in FIG. 3. It can be seen that LA24C11.10 has a blocking effect on the binding of Ang2 to the receptor Tie2.

### Example 6. Phosphorylation Assay of Anti-Ang2 VHH Antibodies

This example demonstrates the inhibitory effect of the anti-Ang2 VHH antibodies of the present invention on hAng2-Fc-induced Tie2 phosphorylation.

The hAng2-induced phosphorylation assay was as follows.

In this research, Expi293 cells overexpressing Tie2, 293-Tie2, were co-incubated with the antibody and a recombinant hAng2-Fc protein, and the content of phosphorylated Tie2 in the system was detected, thereby reflecting the inhibitory effect of different antibodies on the hAng2-fc-induced Tie2 phosphorylation.

The pCHO1.0 vector (Invitrogen) carrying a human Tie2 gene (Beijing Sino Biological, Catalog No. HG10700-M) cloned to a multiple cloning site (MCS) was transfected to Expi-293 cells (Thermo) to produce Expi-293 cells overexpressing human Tie2, that is, 293-Tie2 cells.

The 293-Tie2 cells overexpressing human Tie2 were diluted to 2 × 10⁶ cells/mL and added to a 96-well plate at 100 µL per well, the plate was centrifuged at 400 g for 5 min, and the supernatant was discarded.

An experimental medium was prepared using Expi293 medium (Thermo, Catalog No. A1435102): test antibodies (LA24C11 (24C11) and LA24C11.0 (hz24C11.10) prepared in Example 2, negative control IgG, and positive control Nesvacumab (prepared according to CN202010573625.2)) with initial concentrations of 60 µg/mL were added and then serially diluted in a 1:2 ratio; the final concentration of hAng2-Fc (Beijing Sino Biological: 10691-H02H) was 2.5 µg/mL.

The cells were resuspended with 100 µL of experimental medium per well, and incubated at 37 °C for 15 min.

The medium was removed by centrifugation, and 100 µL of NP-40 lysis buffer (Beyotime, Catalog No. P0013F) containing 1% protease (Thermo, Catalog No. 78442) and a phosphatase inhibitor (Thermo, Catalog No. 78442) was added. The plate was placed on ice for 30 min and centrifuged at 2000 g, and the protein supernatant was collected and stored in a refrigerator at -80 °C.

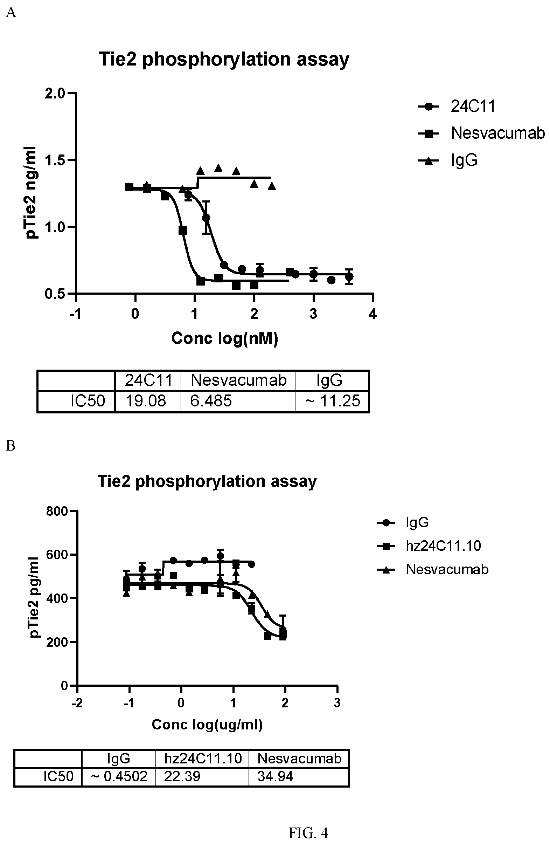

The concentration of pTie2 was detected according to the instruction of a phosphorylated Tie2 ELISA kit (R&D, Catalog No. DYC2720E), and a microplate was coated with a capture antibody in the kit at a concentration of 4 µg/mL and incubated at 4 °C overnight. The plate was washed three times with PBST and blocked with 5% BSA for 1 h. 100 µL of freeze-thawed protein supernatant obtained in the last step and a control pTie2 (R&D, Catalog No. DYC2720E) were added to make a standard curve, and the mixture was incubated at room temperature for 2 h (if the concentration of pTie2 in the sample was too high and exceeded the ELISA detection range, the mixture obtained could be subjected to 2- to 3-fold dilution). The plate was washed three times with PBST, 100 µL of anti-pTyr antibody conjugated with HRP (R&D, Catalog No. DYC2720E) was added, and the mixture was incubated at room temperature for 2 h. The plate was washed 6 times with PBST, 100 µL of TMB (Solarbio, Catalog No. PR1200) was added for color development, and after 15 min, 100 µL of stop buffer (Solarbio, Catalog No. C1058) was added to stop the reaction. OD450-OD620 were measured using a multimode microplate reader SpectraMax i3. The results are shown in FIG. 4A (24C11 is the purified LA24C11 prokaryotic expression supernatant prepared in Example 2) and FIG. 4B.

Therefore, the anti-Ang2 VHH antibodies LA24C11 and LA24C11.10 of the present invention can effectively inhibit hAng2-Fc-induced 293-Tie2 cell phosphorylation *in vitro.*

### Example 7. KDR Reporter Blocking Assay of Anti-VEGF A VHH Antibodies

VEGF A could bind to a related receptor VEGFR2 (KDR), a VEGFR2 signaling pathway was activated, survival, proliferation and migration of vascular endothelial cells were induced, and this research utilized a KDR reporter experiment system and used NFAT-RE-luc2P/KDR HEK293 cells (Promega, Cat. No. CS181401) to detect the blocking effect of the gradiently diluted antibodies on a VEGFA activation-related receptor signaling pathway. Experimental procedures referred to supplier's (Promega) instructions:
NFAT-RE-luc2P/KDR HEK293 cells which were changed into an experimental medium (DMEM medium containing 10% FBS) 3 days in advance were taken out, and the old medium was removed by pipetting. The medium was washed once with PBS, then 1 mL of accutase solution (Sigma, Catalog No. A6964-500ML) was used to digest the cells until the cells became round and were detached from the wall, and 5 mL of diluted medium was used to stop the reaction. The cells were pipetted into a centrifuge tube and centrifuged at 1000 rpm for 5 min, the medium was discarded, 10 mL of diluted medium (DMEM medium containing 10% FBS) was added to resuspend the cells, and the cells were mixed homogeneously and counted. The cell viability should be more than 90%. The cell density was adjusted to 0.8 × 10⁶ cells/mL with the diluted medium, and the mixture was added to a 96-well white cell culture plate at 50 µL/well according to an experimental layout.

A mixed solution of VEGF A with a concentration of 100 ng/mL and the antibodies to be detected which were gradiently diluted was prepared, and the mixture was left to stand for 30 min and added to the 96-well white cell culture plate containing cells at 50 µL/well. The plate was put into a 37 °C incubator with 5% carbon dioxide and incubated for 6 h. The samples to be detected were as follows: negative control IgG; positive control BI-anti-VEGF; LA42F8; LA46E11; LA42F8.5; LA46E11.8; Blank: only containing the diluted medium, no VEGF A and no antibody; VEGF A 100 ng/mL: only containing 100 ng/mL VEGF A.

The 96-well white cell culture plate incubated for 6 h was taken out from the carbon dioxide incubator, and equilibrated for 10-15 min to room temperature. The Bio-Glo Luciferase Assay System equilibrated to room temperature in advance was added to the 96-well white cell culture plate at 100 µL/well according to the experimental layout, and the plate was incubated at room temperature for 5 min in the dark.

Fluorescence values were read using a multimode microplate reader, the chemiluminescence mode was selected for plate reading mode, the end point method was selected for plate reading type, and the wavelength was set to full wavelength. The fluorescence was collected column by column, and the collection time for each column was 1000 ms.

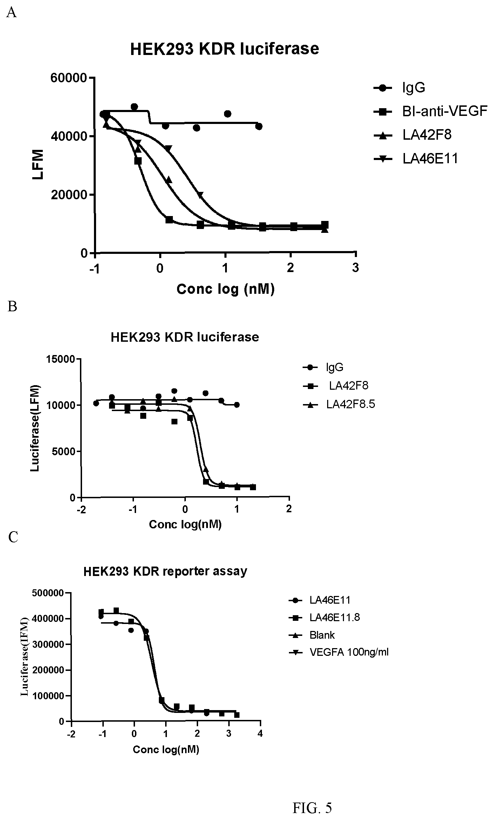

In the experiments performed by the assays described above, the detection results in FIG. 5 show that the anti-VEGF VHH antibodies LA42F8, LA42F8.5, LA46E11, and LA46E11.8 can all block the activation of VEGF A-induced KDR signaling pathways.

### Example 8. Inhibition Assay of VEGF A-Induced HUVEC Survival and Proliferation by Anti-VEGF A VHH

VEGF A could act on VEGFR and other related receptors in vascular endothelial cells, and promote vascular endothelial cell survival, proliferation and migration, thereby inducing neovascularization. This example was based on the VEGF-induced survival and proliferation of human umbilical vein endothelial cells (HUVECs), and the inhibitory effect of the antibodies on the VEGF A-induced survival and proliferation of primary cells was detected. This example determines the survival and proliferation of HUVECs by CCK-8, and the specific method is as follows: HUVECs (Allcells, Catalog No. H-001-CN) were treated one day in advance, and the cells were plated in a 96-well culture plate at 2000 cells/well; the plate was put into in a 37 °C incubator with 5% carbon dioxide and incubated for 24 h.

After the cells were attached to the wall, an experimental medium containing VEGF A with a final concentration of 10 ng/mL and the gradiently diluted antibodies (LA42F8 prepared in Example 2 with an initial concentration of 80 µg/mL and being subjected to serial dilution in a 1:3 ratio, negative control IgG, positive control BI836880, a group (VEGFA) containing only 10 ng/mL of VEGF A, and Blank without VEGF A and antibody) was prepared, the endothelial cell medium in a 96-well plate was replaced by the experimental medium, and the plate was put into a 37 °C incubator with 5% carbon dioxide and incubated for 72 h.

CCK-8 detection solution (Dojindo, Catalog No. CK04) was added at 10 µL/well, and then the plate was put into the 37 °C incubator with 5% carbon dioxide and incubated for 12-24 h.

The absorbance OD₄₅₀-OD₆₂₀ values were read by using a multimode microplate reader.

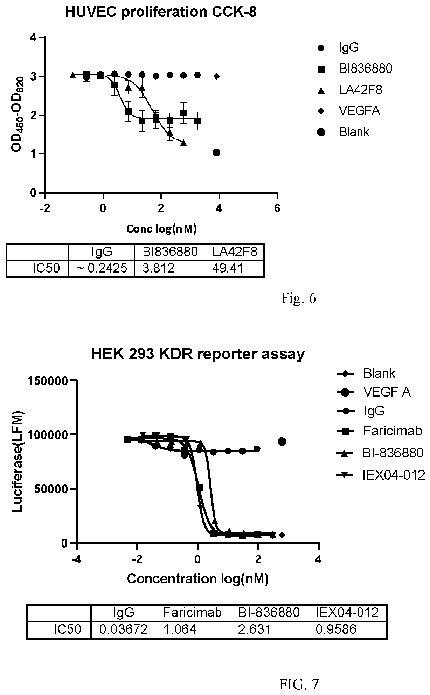

In the experiments performed by the assays described above, the detection results are shown in FIG. 6, from which it can be seen that the anti-VEGFA antibody LA42F8 is able to completely inhibit the VEGFA-induced survival and proliferation of HUVEC cells.

### Example 9. HEK293-KDR Reporter Blocking Assay of Anti-VEGF A/Ang2 Bispecific Antibodies

Chains of the bispecific binding molecules, IEX04-008, IEX04-010 and IEX04-012 (sequences shown in the sequence listing) of the present invention were constructed into pcDNA3.1 vectors, and the bispecific binding molecules were expressed and purified in 293 cells as described in Example 2.

This example detected the blocking effect of the anti-VEGF A/Ang2 bispecific antibodies on VEGF A by HEK293-KDR reporter assay. Experimental procedures referred to those in Example 7.

The binding molecules or controls used were as follows:
Blank: no VEGF A and no antibody;
VEGF A: 100 ng/mL VEGF A;
Negative control IgG: prepared as described above;
Positive control Faricimab: prepared as described above, with an initial concentration of 13.5 µg/mL and being subjected to gradient dilution in a 1:3 ratio;
Positive control BI-836880: prepared as described above, with an initial concentration of 13.5 µg/mL and being subjected to gradient dilution in a 1:3 ratio; and
IEX04-012: prepared as described above, with an initial concentration of 13.5 µg/mL and being subjected to gradient dilution in a 1:3 ratio.

The detection results in FIG. 7 show that the bispecific binding molecule IEX04-012 inhibits the activation of VEGF-induced KDR signaling pathways, and has better inhibitory ability compared with the control antibody BI836880.

### Example 10. HUVEC Proliferation Inhibition Assay of Anti-VEGF A/Ang2 Bispecific Binding Molecules

In this research, the inhibitory effect of the anti-VEGF A/Ang2 bispecific binding molecules on the VEGF A-induced survival and proliferation of HUVEC cells was detected by HEK293-KDR reporter assay.

The experimental procedures were as those in Example 8, and the antibodies used were as follows:
Panel A: IEX04-008, negative control IgG, positive controls Faricimab and BI836880, Blank (i.e., no antibody and VEGF A), and VEGF A group (i.e., only 20 ng/mL of VEGFA added) prepared as described above with initial concentrations of 20 µg/mL and being subjected to serial dilution in a 1:3 ratio;
Panel B: negative control IgG, BI-anti-VEGF, IEX04-010, Blank (i.e., no antibody and VEGF A), and VEGF A group (i.e., only 20 ng/mL of VEGFA added) prepared as described above with initial concentrations of 80 µg/mL and being subjected to serial dilution in a 1:3 ratio; and
Panel C: IEX04-012, negative control IgG, positive controls Faricimab and BI836880, Blank (i.e., no antibody and VEGF A), and VEGF A group (i.e., only 20 ng/mL of VEGFA added) prepared as described above with initial concentrations of 20 nM and being subjected to serial dilution in a 1:3 ratio.

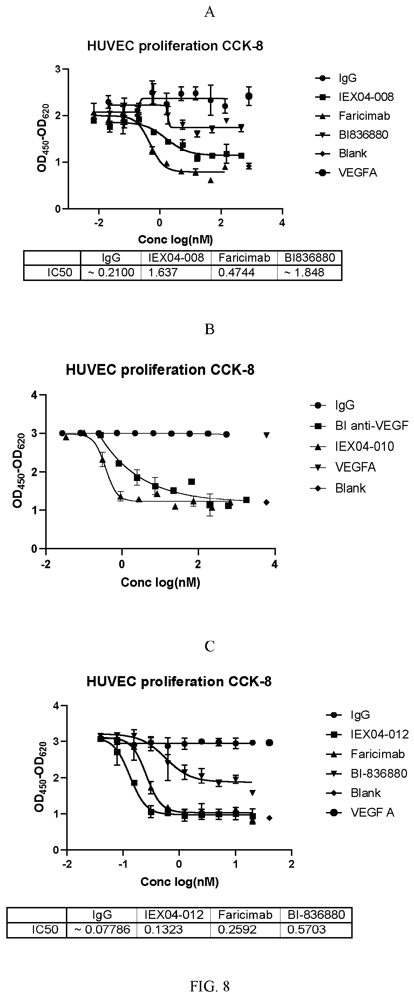

The detection results in FIG. 8 show that the bispecific binding molecules IEX04-008, IEX04-010 and IEX04-012 all inhibit VEGF-induced survival and proliferation of HUVEC cells, and IEX04-012 has lower IC₅₀ and better inhibitory ability compared with the control antibodies BI836880 and Faricimab.

### Example 11. Ang2 Blocking Assay of Anti-VEGF A/Ang2 Bispecific Antibodies

Ang2 could bind to its native receptor Tie2, and this research detected the blocking effect of the anti-VEGF A/Ang2 bispecific binding molecules on the binding between Ang2 and Tie2 by ELISA assay and FACS assay.

### (1) ELISA

The ability of IEX04-008, IEX04-010, IEX04-012, and control antibodies BI-836880 and Faricimab to block the binding of human Ang2 to hTie2 was detected by ELISA.

The hTie2 protein (Beijing Sino Biological) was resuspended and dissolved to a concentration of 2 µg/mL with PBS, and a microplate was coated with the protein and incubated overnight. The plate was blocked with 5% BSA for 1 h, and a biotinylated antigen (Recombinant Biotinylated hAngiopoietin-2 protein (R&D)) was diluted to 600 µg/mL and added to the plate at 50 µL/well. The antibodies prepared as described above (IEX04-008, IEX04-010, IEX04-012, positive control antibodies BI836880 and Faricimab, and negative control IgG) were each diluted serially in a 1:2 ratio starting from the maximum concentration of 300 nM for a total of 8 or 12 dilution gradients. The diluted antibodies were added at 50 µL/well and incubated in PBS on ice for 30 min, and the biotinylated antigen had a final concentration of 300 ng/mL. The antigen-antibody mixture obtained as described above was incubated in the microplate for 90 min and washed three times with PBS, and the supernatant was discarded. 100 µL of Avidin-HRP (Invitrogen) diluted in a 1:10000 ratio was added to each well, incubated at room temperature for 30 min, and washed six times with PBS. A TMB chromogenic solution (solarbio) was added to the plate at 100 µL/well for color development for 1 min, and the reaction was stopped by adding a stop solution (Solarbio) at 100 µL/well.

The plate was read on a microplate reader at OD₄₅₀ and OD₆₂₀ per well.

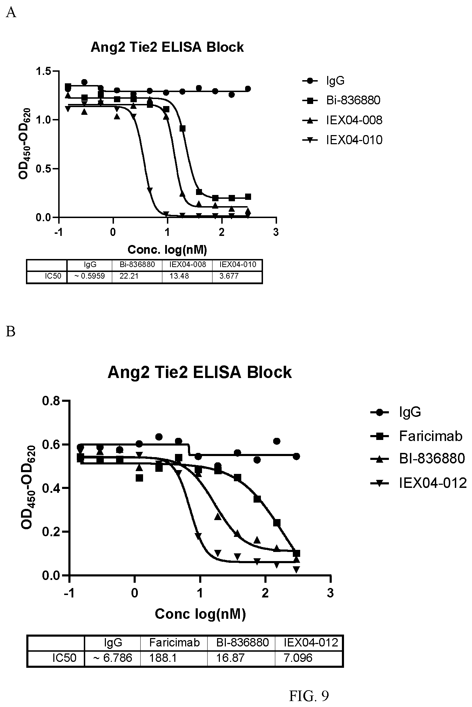

The results (see FIG. 9) show that IEX04-008, IEX04-010, IEX04-012, and the control antibody BI836880 all have a complete blocking effect, and that IC₅₀ of the bispecific binding molecules of the present invention are significantly smaller than that of the positive control antibody.

### (2) Flow cytometry assay (FACS)

The ability of IEX04-008, IEX04-010, IEX04-012, positive control antibodies BI-836880 and Faricimab, and negative control IgG to block the binding of human Ang2-hFc to Tie2 on the cell surface was detected by FACS.

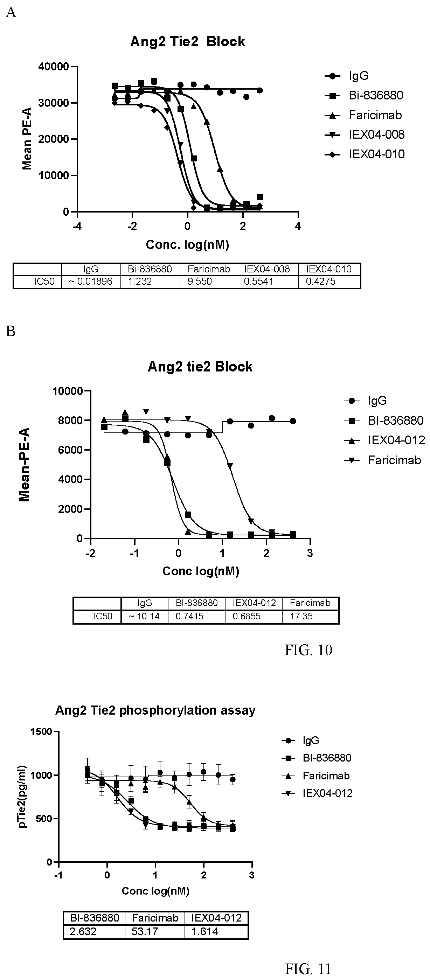

The antigen hAng2-Fc protein (Beijing Sino Biological, Catalog No. 10691-H02H) was diluted to 4 µg/mL and added at 50 µL/well. The antibodies prepared as described above (IEX04-008, IEX04-010, IEX04-012, positive control antibodies BI-836880 and Faricimab, and negative control IgG) were each 2-fold diluted gradiently starting from the maximum concentration of 800 nM for a total of 12 dilution gradients. The diluted antibodies were added at 50 µL/well and incubated in PBS on ice for 30 min, the antigen hAng2-Fc protein had a final concentration of 2 µg/mL, and each antibody had a maximum final concentration of 400 nM. 293-Tie2 cells prepared as described above were adjusted to 2 × 10⁵ cells/well and added at 100 µL/well. The cells were centrifuged at 300 g for 5 min. The supernatant was discarded. Then the cells were resuspended in an antigen-antibody mixture. The mixture was incubated on ice for 30 min. PBS was added at 100 µL/well. After centrifugation at 300 g for 5 min, the mixture was washed once with PBS. 100 µL of goat anti-human IgG-PE (SouthernBiotech) diluted in a 1:200 ratio was added to each well. After a 20-min ice bath, PBS was added at 100 µL/well. The mixture was centrifuged at 300 g for 5 min, prior to one wash with PBS. The cells were resuspended with 100 µL of PBS. The cell fluorescence signal values were measured by a flow cytometer (BD Biosciences). According to MFI, the concentration-dependent curve was fitted with GraphPad. The results are shown in FIG. 10. It is shown that the bispecific binding molecules IEX04-008, IEX04-010, and IEX04-012 can all effectively block the binding of human Ang2-hFc to Tie2, and have IC₅₀ lower than that of the positive control.

### Example 12. Ang2 Phosphorylation Inhibition Assay of Anti-VEGF A/Ang2 Bispecific Binding Molecules

This example demonstrates the inhibitory effect of the bispecific binding molecules of the present invention on hAng2-Fc-induced Tie2 phosphorylation by using hAng2-induced phosphorylation assay.

In this research, Expi293 cells overexpressing Tie2, 293-Tie2, were co-incubated with the bispecific binding molecules and a recombinant hAng2-Fc protein, and the content of phosphorylated Tie2 in the system was detected, thereby reflecting the inhibitory effect of different antibodies on the hAng2-Fc-induced Tie2 phosphorylation.

The overexpressed 293-Tie2 cells prepared above were diluted to 2 × 10⁶ cells/mL and added to a 96-well plate at 100 µL per well, the plate was centrifuged at 400 g for 5 min, and the supernatant was discarded.

An experimental medium was prepared using Expi293 medium (Thermo, Catalog No. A1435102), in which test antibodies (IEX04-012 prepared above, positive controls BI-836880 and Faricimab, and negative control IgG) with maximum final concentration of 60 µg/mL were each added and then serially diluted in a 1:2 ratio; the final concentration of hAng2-Fc (Beijing Sino Biological, Catalog No. 10691-H02H) was 2.5 µg/mL.

The cells were resuspended with 100 µL of the experimental medium per well and incubated at 37 °C for 15 min. The medium was removed by centrifugation. 100 µL of NP-40 lysis buffer containing 1% protease and a phosphatase inhibitor was added, and the plate was left to stand on ice for 30 min. The plate was centrifuged at 2000 g, and the protein supernatant was collected and stored in a refrigerator at -80 °C.

The concentration of pTie2 was detected according to the instruction of a phosphorylated Tie2 ELISA kit (R&D, DYC2720E), and a microplate was coated with a capture antibody at a concentration of 4 µg/mL and incubated at 4 °C overnight. The plate was washed three times with PBST and blocked with 5% BSA for 1 h. 100 µL of the sample to be detected and a control pTie2 (R&D, DYC2720E) were added to make a standard curve, and the mixture was incubated at room temperature for 2 h (if the concentration of pTie2 in the sample was too high and exceeded the ELISA detection range, the mixture obtained could be subjected to 2- to 3-fold dilution). The plate was washed three times with PBST, 100 µL of anti-pTyr antibody conjugated with HRP (R&D, Catalog No. DYC2720E) was added, and the mixture was incubated at room temperature for 2 h. The plate was washed 6 times with PBST, 100 µL of TMB was added for color development, and after 15 min, 100 µL of stop buffer was added to stop the reaction. OD450-OD620 of each well was measured using a spectrophotometer.

The results are shown in FIG. 11. The antibody IEX04-012 of the present invention can effectively inhibit hAng2-Fc-induced 293-Tie2 phosphorylation *in vitro,* and IC₅₀ is superior to that of the positive control.

### Example 13. Inhibition Assay of Ang2 Vascular Endothelial Cell Leakage by Anti-VEGF A/Ang2 Bispecific Binding Molecules

This research identified the effect and function of anti-VEGF A/Ang2 bispecific binding molecules on vascular endothelial cell leakage by HUVEC-Tie2 leakage assay.

HUVEC-Tie2 cells overexpressing Tie2 were obtained by transfecting HUVEC cells (Allcells, Catalog No. H-001-CN) with lentivirus.

300 µL of EGM-2 medium was plated on the bottom layer of a mini-well 96-well insert culture dish, the cells were digested with accutase (Sigma) and HUVEC-Tie2 was obtained. The cells were resuspended to 1 × 10⁷ cells/mL using the EGM-2 medium and plated at 100 µL/well on the top layer of the culture dish. The lower chamber medium (EGM-2 medium) was replaced every 24 h, and after 24 h, the lower chamber medium was replaced by an experimental medium, wherein the experimental medium contained the following components:
Blank: EGM-2 medium (Lonza, Catalog No. CC-5035);
VEGF A group: EGM-2 Medium + 20 ng/mL VEGF (R&D, Catalog No. 293-VE);
IgG group (VEGF A + IgG): EGM-2 medium + 20 ng/mL VEGF + 10 µg/mL IgG;
Ang 1 group (VEGF A + Ang1): EGM-2 medium + 20 ng/mL VEGF (R&D, Catalog No. 293-VE) + 200 ng/mL Ang1 (R&D, Catalog No. 923-AN);
IEX04-012 group (VEGF A + IEX04-012): EGM-2 medium + 20 ng/mL VEGF + 10 µg/mL IEX04-012;
BI-836880 group (VEGF A + BI-836880): EGM-2 medium + 20 ng/mL VEGF + 10 µg/mL BI-836880; and
Faricimab group (VEGF A + Faricimab): EGM-2 medium + 20 ng/mL VEGF + 10 µg/mL Faricimab.

The experimental medium described above was placed and incubated at 37 °C with 5% CO₂.

After 24 h, 1 µL of FITC-Dextran (Sigma, Catalog No. FD2000S-1G) (4 mg/mL) was added to the experimental medium per well. The mixture was placed at 37 °C with 5% CO₂. After 30 min, the lower chamber medium was taken out, diluted in a 1:10 ratio with PBS, and detected by a multimode microplate reader at an exciting wavelength of 488 nm and an emitting wavelength of 535 nm.

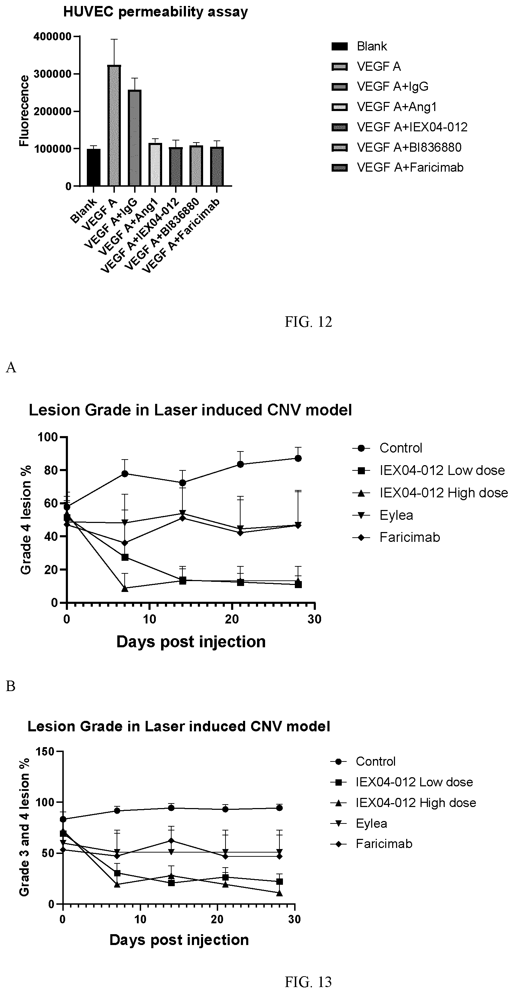

The results are shown in FIG. 12, showing that the antibody IEX04-012 of the present invention can effectively reduce the VEGF-induced vascular endothelial cell permeability.

### Example 14. Laser-Induced Choroidal Neovascularization Pharmacodynamic Test

This assay adopted a rhesus monkey laser-induced choroidal neovascularization model to determine the neovascularization resistance of the bispecific binding molecule IEX04-012 of the present invention.

### Rhesus monkey:

Species: a rhesus monkey; grade: a normal grade; weight: 3.30-4.20 kg of body weight when purchased, and 3.35-4.35 kg of body weight during molding; source: Sichuan Hengshu Bio-Technology; production license number: SCXK (Sichuan) 2019-029; laboratory animal quality certification number: No.0023356.

In this test, laser photocoagulation was performed around the macular fovea of the fundus of the rhesus monkey to induce the choroidal neovascularization in the fundus and establish an animal model similar to the human choroidal neovascularization. Before and after 20 days of photocoagulation, fundus fluorescence angiography was performed to judge the molding condition. 20 rhesus monkeys (half male and half female) which were successfully molded were selected to be divided into 5 groups, namely a model control group, an IEX04-012 low-dose group, an IEX04-012 high-dose group, an Elyea group, and a Faricimab group, wherein each group contained 4 monkeys, half male and female.

On day 21 after photocoagulation, each group of monkeys was administered individually according to the doses in the table. IEX04-012, Eylea (Bayer), or Faricimab (all dissolved in 0.9% sodium chloride injectable solution) was administered by intravitreal injection at both eyes, and the model control was given an equal volume of 0.9% sodium chloride injectable solution. Color fundus photography, fundus fluorescence angiography (leak spot statistics and leak area measurements) (Robin J Goody, Wenzheng Hu, Afshin Shafiee et al., Optimization of laser-induced choroidal neovascularization in African green monkeys. Experimental Eye Research, Exp Eye Res. 2011 92(6):464-72), and optical coherence tomography (OCT, Wang Q, Lin X, Xiang W et al., Assessment of laser induction of Bruch's membrane disruption in monkey by spectral-domain optical coherence tomography. British Journal of Ophthalmology, 2015, 99(1):119-24) were performed on each group of animals on day 7, 14, 21, and 28 after the administration to observe the inhibition effect of the test sample on the choroidal neovascularization. The animals were euthanized on day 29 after the administration, and then both eyes were taken out for histological examination with immunohistochemical (HE) staining.

### Experimental design table

| Group Control | Dose / | Administration volume / | Concentration X mg/ml | Route of administration |
|---|---|---|---|---|
| IEX04-012 Low dose | 10 µg/eye | 50 µL/eye | 0.2mg/ml | Intravitreal administration |
| IEX04-012 High dose | 30 µg/eye | 50 µL/eye | 0.6mg/ml | Intravitreal administration |
| Eylea | 10 µg/eye | 50 µL/eye | 0.2mg/ml | Intravitreal administration |
| Faricimab | 30 µg/eye | 50 µL/eye | 0.6mg/ml | Intravitreal administration |

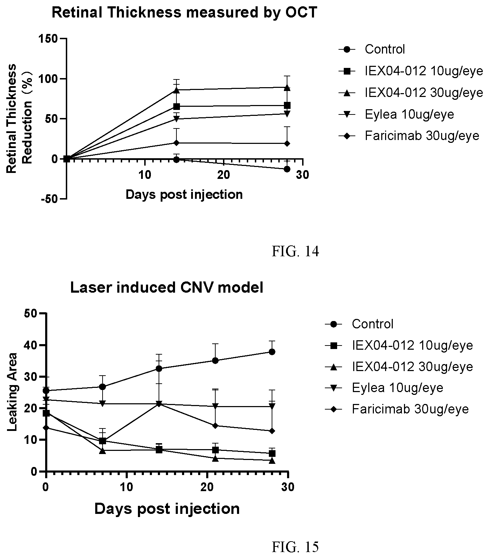

The results in FIGs. 13-15 show that the bispecific binding molecule of the present invention showed significant anti-neovascularization after 28 days of administration, and the statistics of the number of grade four leakage spots (FIG. 13A) and the number of grade three to four leakage spots (FIG. 13B) showed that the treated animals of the IEX04-012 group had significantly fewer high-leakage spots than those of the control and positive control administration groups. The OCT results showed a significant decrease in retinal thickness in the animals of the IEX04-012 treatment group, suggesting that the retinal edema degree is reduced and the effect is superior to that of the control (FIG. 14). The fundus fluorescence angiographic results show that the fundus leakage area of the IEX04-012 treatment group was significantly reduced, and the effect was superior to those of positive controls Eylea and Faricimab (FIG. 15), indicating that the antibody of the present invention can significantly inhibit the leakage caused by neovascularization. In conclusion, it is verified that the antibody of the present invention in combination with the anti-VEGF inhibitor has a significant inhibitory effect on the laser-induced fundus neovascularization, and has the function of protecting the integrity of blood vessels.

29 days after the administration, rhesus monkeys were anesthetized with pentobarbital sodium according to the body weight (at about 30 mg/kg by intravenous injection, and the dose could be adjusted according to health conditions of the animals) and euthanized by bleeding from abdominal aorta or femoral artery. The rhesus monkeys were roughly observed, and bilateral eyeballs were taken.

Some animal eyes were fixed in a modified Davidson's fixative solution and embedded in paraffin, and laser-molded areas were selected for conventional HE staining such as CD31 IHC staining to perform the histopathological examination.

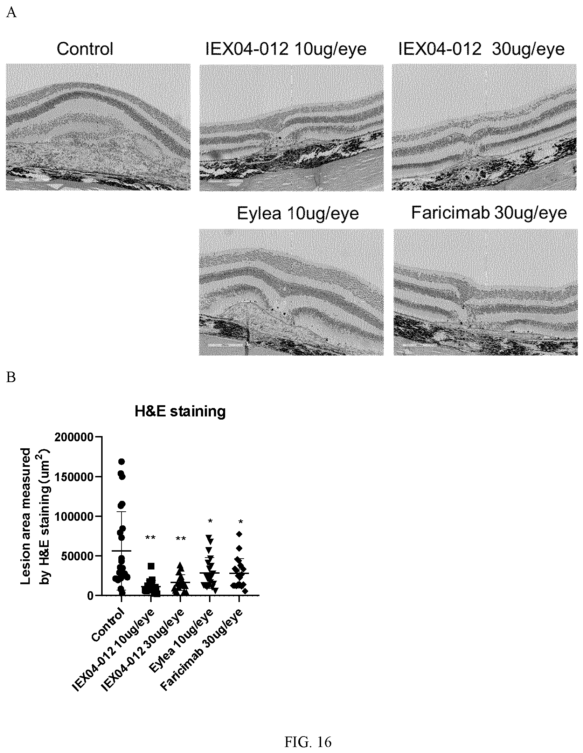

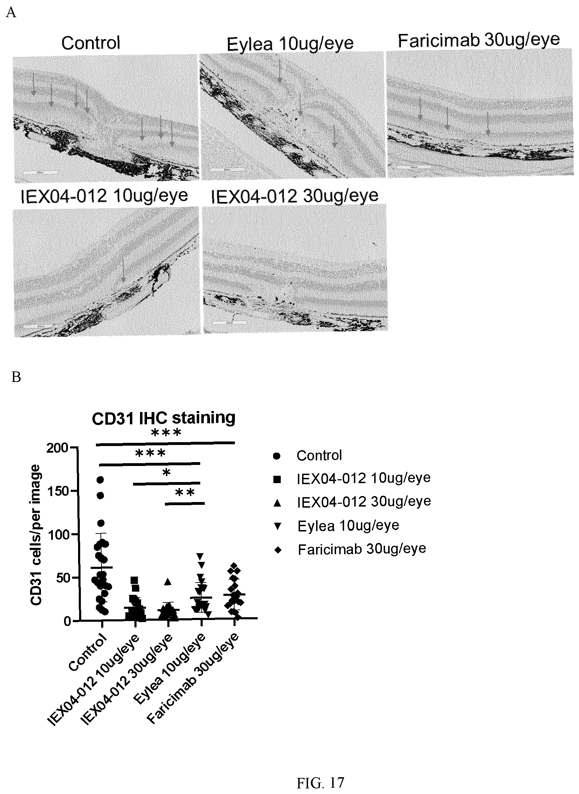

In the pathological section of the antibody group of the present invention, the retinopathy area was significantly reduced, the retinal edema was alleviated, and the tissue hyperplasia of the laser injury area was reduced compared with the anti-VEGF single treatment. The results show better retina morphological improvement (see FIG. 16), the inhibition of the retinochoroidal neovascularization, and the enhancement of the blood vessel integrity function (FIG. 17).

### SEQUENCE LISTING

| SEQ ID NO | Antibody name | LA42F8 (VEGFA VHH) |
|---|---|---|
| 1 | HCDR1 | GFNLDYYPIG |
| 2 | HCDR2 | CISSVGSTNYADSVKG |
| 3 | HCDR3 | DPLCSALILPPPFLS |
| 4 | VH | QLQLVESGGGLVQPGGSLRLSCAASGFNLDYYPIGWFRQAPGKEREGVSCISS VGSTNYADSVKGRFTISRDNAKNTVYLQMNSLKPEDTAVYYCAADPLCSALIL PPPFLSWGQGTQVTVSS |
| | Antibody name | LA42F8.5 |
| 1 | HCDR1 | GFNLDYYPIG |
| 2 | HCDR2 | CISSVGSTNYADSVKG |
| 3 | HCDR3 | DPLCSALILPPPFLS |
| 5 | VH | EVQLLESGGGLVQPGGSLRLSCAASGFNLDYYPIGWFRQAPGKGLEGVSCISS VGSTNYADSVKGRFTISRDNSKNTVYLQMNSLRAEDTAVYYCAADPLCSALIL PPPFLSWGQGTQVTVSS |
| | Antibody name | LA46E11 (VEGFA VHH) |
| 6 | HCDR1 | GSIFSINAMG |
| 7 | HCDR2 | TMIGGSSTFYADSVNG |
| 8 | HCDR3 | DVTGRSGTSIWNKRDDY |
| 9 | VH | QVQLEESGGGLVQPGGSLRLSCVASGSIFSINAMGWHRQAPGSQRELVATMIG GSSTFYADSVNGRFTISRDNAKNTVYLQMNSLKLEDTAVYYCYADVTGRSGTS IWNKRDDYWGQGTLVTVSS |
| | Antibody name | LA46E11.8 (VEGFA VHH) |
| 6 | HCDR1 | GSIFSINAMG |
| 10 | HCDR2 | TMIGGSSTFYAESVQG |
| 8 | HCDR3 | DVTGRSGTSIWNKRDDY |
| 11 | VH | EVQLLESGGGLVQPGGSLRLSCAASGSIFSINAMGWHRQAPGKQRELVATMIG GSSTFYAESVQGRFTISRDNSKNTVYLQMNSLRAEDTAVYYCYADVTGRSGTSI WNKRDDYWGQGTQVTVSS |
| | Antibody name | LA24C11 (Ang2 VHH) |
| 16 | HCDR1 | GFALDYYAIG |
| 17 | HCDR2 | CISSGDGSTYYADSVKG |
| 18 | HCDR3 | DSRGDDVACEGLRRNEYDY |
| 19 | VH | QVQLVESGGGLVQPGGSLRLSCAASGFALDYYAIGWFRQAPGKEREGVSCISS GDGSTYYADSVKGRFTISRDNAKNTVYLQMNSLKPEDTAVYYCATDSRGDDV ACEGLRRNEYDYWGQGTQVTVSS |
| | Antibody name | LA24C11.10 (Ang2 VHH) |
| 16 | HCDR1 | GFALDYYAIG |
| 20 | HCDR2 | CISSGEGSTYYADSVKG |
| 18 | HCDR3 | DSRGDDVACEGLRRNEYDY |
| 21 | VH | EVQLLESGGGLVQPGGSLRLSCAASGFALDYYAIGWFRQAPGKGLEGVSCISSGEGS TYYADSVKGRFTISRDNSKNTVYLQMNSLRAEDTAVYYCATDSRGDDVACEGLRRNE YDYWGQGTLVTVSS |
| | Antibody name | IEX04-008 LA46E11.8 (VHH) (underlined) + linker (bold) + LA46E11.8 (VHH) (underlined) + LA24C11.10 (italic) |
| 22 | Full length | EVQLLESGGGLVQPGGSLRLSCAASGSIFSINAMGWHRQAPGKQRELVATMIG GSSTFYAESVQGRFTISRDNSKNTVYLQMNSLRAEDTAVYYCYADVTGRSGTSI WNKRDDYWGQGTQVTVSSGGGGSGGGGSEVQLLESGGGLVQPGGSLRLSC AASGSIFSINAMGWHRQAPGKQRELVATMIGGSSTFYAESVQGRFTISRDNSKN TVYLQMNSLRAEDTAVYYCYADVTGRSGTSIWNKRDDYWGQGTQVTVSSGG GGSGGGGSEVQLLESGGGLVQPGGSLRLSCAASGFALDYYAIGWFRQAPGKGLEG VSCISSGEGSTYYADSVKGRFTISRDNSKNTVYLQMNSLRAEDTAVYYCATDSRGDDV ACEGLRRNEYDYWGQGTLVTVSS |
| | Antibody name | IEX04-010 VEGFR extracellular domain + Fc + linker (underlined) + LA24C11.10 (italic) |
| 24 | Full length | SDTGRPFVEMYSEIPEIIHMTEGRELVIPCRVTSPNITVTLKKFPLDTLIPDGKRII WDSRKGFIISNATYKEIGLLTCEATVNGHLYKTNYLTHRQTNTIIDVVLSPSHGI ELSVGEKLVLNCTARTELNVGIDFNWEYPSSKHQHKKLVNRDLKTQSGSEMK KFLSTLTIDGVTRSDQGLYTCAASSGLMTKKNSTFVRVHEKDKTHTCPPCPAPE LLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKSLSLSP GGGGGSGGGGSGGGGSEVQLLESGGGLVQPGGSLRLSCAASGFALDYYAIGWFR QAPGKGLEGVSCISSGEGSTYYADSVKGRFTISRDNSKNTVYLQMNSLRAEDTAVYY CATDSRGDDVACEGLRRNEYDYWGQGTLVTVSS* |
| | Name | Aflibercept (VEGFR extracellular domain (underlined) + Fc (italic + bold), wherein FLT1 domain 2 is underlined, and KDR domain 3 is underlined and bold) |
| 25 | Full length | SDTGRPFVEMYSEIPEIIHMTEGRELVIPCRVTSPNITVTLKKFPLDTLIPDGKRII WDSRKGFIISNATYKEIGLLTCEATVNGHLYKTNYLTHRQTNTIIDVVLSPSHGI ELSVGEKLVLNCTARTELNVGIDFNWEYPSSKHQHKKLVNRDLKTQSGSE MKKFLSTLTIDGVTRSDQGLYTCAASSGLMTKKNSTFVRVHEKDKTHTCP PCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQP ENNYKTTPPVLDSDGSFFLYSKLTVDKSRWQQGNVFSCSVMHEALHNHYTQK SLSLSPG |
| 26 | VEGFR extracellular domain | SDTGRPFVEMYSEIPEIIHMTEGRELVIPCRVTSPNITVTLKKFPLDTLIPDGKRII WDSRKGFIISNATYKEIGLLTCEATVNGHLYKTNYLTHRQTNTIIDVVLSPSHGI ELSVGEKLVLNCTARTELNVGIDFNWEYPSSKHQHKKLVNRDLKTQSGSEMK KFLSTLTIDGVTRSDQGLYTCAASSGLMTKKNSTFVRVHEK |
| 27 | Fc | DKTHTCPPCPAPELLGGPSVFLFPPKPKDTLMISRTPEVTCVVVDVSHEDPEVK FNWYVDGVEVHNAKTKPREEQYNSTYRVVSVLTVLHQDWLNGKEYKCKVS NKALPAPIEKTISKAKGQPREPQVYTLPPSRDELTKNQVSLTCLVKGFYPSDIAV EWESNGQPENNYKTTPPVLDSDGSFFLYSKLTVDKSRWQQGNVFSCSVMHEA LHNHYTQKSLSLSPG |
| \ | Antibody name | IEX04-012 Ranibizumab scFv containing disulfide bond mutations + linker (bold) + LA24C11.10 (italic) |
| 28 | Full length | DIQLTQSPSSLSASVGDRVTITCSASQDISNYLNWYQQKPGKAPKVLIYFTSSLH SGVPSRFSGSGSGTDFTLTISSLQPEDFATYYCQQYSTVPWTFGCGTKVEIKGG GGSGGGGSGGGGSGGGGSEVQLVESGGGLVQPGGSLRLSCAASGYDFTHYG MNWVRQAPGKCLEWVGWINTYTGEPTYAADFKRRFTFSLDTSKSTAYLQMN SLRAEDTAVYYCAKYPYYYGTSHWYFDVWGQGTLVTVSSGGGGSGGGGSG GGGSEVQLLESGGGLVQPGGSLRLSCAASGFALDYYAIGWFRQAPGKGLEGVSCIS SGEGSTYYADSVKGRFTISRDNSKNTVYLQMNSLRAEDTAVYYCATDSRGDDVACEG LRRNEYDYWGQGTLVTVSS* |
| | | Ranibizumab scFv containing disulfide bond mutations |
| 29 | scFv | DIQLTQSPSSLSASVGDRVTITCSASQDISNYLNWYQQKPGKAPKVLIYFTSSLH SGVPSRFSGSGSGTDFTLTISSLQPEDFATYYCQQYSTVPWTFGCGTKVEIKGG GGSGGGGSGGGGSGGGGSEVQLVESGGGLVQPGGSLRLSCAASGYDFTHYG MNWVRQAPGKCLEWVGWINTYTGEPTYAADFKRRFTFSLDTSKSTAYLQMN SLRAEDTAVYYCAKYPYYYGTSHWYFDVWGQGTLVTVSS DIQLTQSPSSLSASVGDRVTITCSASQDISNYLNWYQQKPGKAPKVLIYFTSSLH SGVPSRFSGSGSGTDFTLTISSLQPEDFATYYCQQYSTVPWTFGCGTKVEIK |
| 30 | VL | DIQLTQSPSSLSASVGDRVTITCSASQDISNYLNWYQQKPGKAPKVLIYFTSSLH SGVPSRFSGSGSGTDFTLTISSLQPEDFATYYCQQYSTVPWTFGCGTKVEIK |
| 31 | LCDR1 | QDISNYLN |
| 32 | LCDR2 | FTSSLHS |
| 33 | LCDR3 | QQYSTVPWT |
| 34 | VH | EVQLVESGGGLVQPGGSLRLSCAASGYDFTHYGMNWVRQAPGKCLEWVGWI NTYTGEPTYAADFKRRFTFSLDTSKSTAYLQMNSLRAEDTAVYYCAKYPYYYG TSHWYFDVWGQGTLVTVSS |
| 35 | HCDR1 | GYDFTHY |
| 36 | HCDR2 | NTYTGE |
| 37 | HCDR3 | YPYYYGTSHWYFDV |
| 23 | Linker | (GGGGS)ₙ, wherein n = 1, 2, 3, or 4 |
| | | Positive control antibody BI836880 (VEGFA terminal) |
| 12 | VH | DVQLVESGGGLVQPGGSLRLSCAASGRTFSSYSMGWFRQAPGKEREFVVAISK GGYKYDAVSLEGRFTISRDNAKNTVYLQINSLRPEDTAVYYCASSRAYGSSRLR LADTYEYWGQGTLVTVSS |
| | | Positive control antibody Faricimab |
| 13 | HC 1 | EVQLVESGGGLVQPGGSLRLSCAASGYDFTHYGMNWVRQAPGKGLEWVGWI NTYTGEPTYAADFKRRFTFSLDTSKSTAYLQMNSLRAEDTAVYYCAKYPYYYG TSHWYFDVWGQGTLVTVSSASTKGPSVFPLAPSSKSTSGGTAALGCLVKDYFP EPVTVSWNSGALTSGVHTFPAVLQSSGLYSLSSVVTVPSSSLGTQTYICNVNHK PSNTKVDKKVEPKSCDKTHTCPPCPAPEAAGGPSVFLFPPKPKDTLMASRTPEV TCVVVDVSHEDPEVKFNWYVDGVEVHNAKTKPREEQYNSTYRVVSVLTVLA QDWLNGKEYKCKVSNKALGAPIEKTISKAKGQPREPQVYTLPPCRDELTKNQ VSLWCLVKGFYPSDIAVEWESNGQPENNYKTTPPVLDSDGSFFLYSKLTVDKSR WQQGNVFSCSVMHEALHNAYTQKSLSLSPGK |
| 40 | LC1 | DIQLTQSPSSLSASVGDRVTITCSASQDISNYLNWYQQKPGKAPKVLIYFTSSLH SGVPSRFSGSGSGTDFTLTISSLQPEDFATYYCQQYSTVPWTFGQGTKVEIKRTV AAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC |
| 14 | HC 2 | QVQLVQSGAEVKKPGASVKVSCKASGYTFTGYYMHWVRQAPGQGLEWMG WINPNSGGTNYAQKFQGRVTMTRDTSISTAYMELSRLRSDDTAVYYCARSPNP YYYDSSGYYYPGAFDIWGQGTMVTVSSASVAAPSVFIFPPSDEQLKSGTASVV CLLNNFYPREAKVQWKVDNALQSGNSQESVTEQDSKDSTYSLSSTLTLSKAD YEKHKVYACEVTHQGLSSPVTKSFNRGECDKTHTCPPCPAPEAAGGPSVFLFPP KPKDTLMASRTPEVTCVVVDVSHEDPEVKFNWYVDGVEVHNAKTKPREEQY NSTYRVVSVLTVLAQDWLNGKEYKCKVSNKALGAPIEKTISKAKGQPREPQV CTLPPSRDELTKNQVSLSCAVKGFYPSDIAVEWESNGQPENNYKTTPPVLDSDG SFFLVSKLTVDKSRWQQGNVFSCSVMHEALHNAYTQKSLSLSPGK |
| 41 | LC2 | SYVLTQPPSVSVAPGQTARITCGGNNIGSKSVHWYQQKPGQAPVLVVYDDSDR PSGIPERFSGSNSGNTATLTISRVEAGDEADYYCQVWDSSSDHWVFGGGTKLTV LSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSC |
| | | Positive control antibody BI836880 (VEGFA/HSA/Ang2) |
| 15 | VH | DVQLVESGGGLVQPGGSLRLSCAASGRTFSSYSMGWFRQAPGKEREFVVAISK GGYKYDAVSLEGRFTISRDNAKNTVYLQINSLRPEDTAVYYCASSRAYGSSRLR LADTYEYWGQGTLVTVSSGGGGSGGGSEVQLVESGGGLVQPGNSLRLSCAA SGFTFSSFGMSWVRQAPGRGLEWVSSISGSGSDTLYADSVKGRFTISRDNAKTT LYLQMNSLRPEDTAVYYCTIGGSLSRSSQGTLVTVSSGGGGSGGGSEVQLVES GGGLVQPGGSLRLSCAVSGITLDDYAIGWFRQAPGKEREGVSAIRSSGGSTYYA DSVKGRFTISSDNSKNTVYLQMNSLRPEDTAVYYCAAVPAGRLRYGEQWYPIY EYDAWGQGTLVTVSS |
| | | Negative control IgG |
| 38 | Heavy chain | EVRLLESGGGLVQPGGSLRLSCAASGFTFSNYAMGWVRQAPGKGLEWVSAIS GSGGSTYYADSVKGRFTTSRDDSKNALYLQMNSLRAEDTAVYYCARGGPGW YAADVWGQGTTVTVSSASTKGPSVFPLAPSSKSTSGGTAALGCLVKDYFPEPV TVSWNSGALTSGVHTFPAVLQSSGLYSLSSVVTVPSSSLGTQTYICNVNHKPSN TKVDKKAEPKSCDKTHTCPPCPAPELLGGPSVFLFPPKPKDTLMISRTPEVTCV VVDVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQVSLTC LVKGFYPSDIAVEWESNGQPENNYKTTPPVLDSDGSFFLYSKLTVDKSRWQQG NVFSCSVMHEALHNHYTQKSLSLSPGK |
| 42 | Light Chain | DIQMTQSPSSLSASVGDRVTITCRASQSISSYLNWYQQKPGKAPKLLIYAASSL QSGVPSRFSGSGSGTDFTLTISSLQPEDFATYYCQQADLPAFAFGGGTKVEIKRT VAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC |
| | | Positive control antibody BI-anti-VEGF |
| 39 | | DVQLVESGGGLVQPGGSLRLSCAASGRTFSSYSMGWFRQAPGKEREFVVAISK GGYKYDAVSLEGRFTISRDNAKNTVYLQINSLRPEDTAVYYCASSRAYGSSRLR LADTYEYWGQGTLVTVSS |

### Example 15. Formula determination experiment of bispecific antibody of the present invention

The effect of the excipients (sodium dihydrogen phosphate (monohydrate), disodium hydrogen phosphate (heptahydrate), trehalose, histidine, methionine, proline, glycine, sucrose, hydroxypropyl-β-cyclodextrin, and polysorbate 80) on the stability of IEX04-012 protein was examined in this experiment, and the detailed formula information is shown in Table 5.

### Materials:

The information on the excipients and/or reagents used in the formula study of the present invention is given in Table 4 below.

**Table 4. Reagent information**

| Name | Manufacturer & brand | Cat# |
|---|---|---|
| Histidine | Ajinomoto, Shanghai | N/A |
| Histidine | Ajinomoto, Shanghai | |
| | Merck, Germany | 1.04352.1000 |
| | Merck, Germany | |
| Sodium dihydrogen phosphate (monohydrate) | Merck, Germany | 1.06349.1000 |
| Sodium dihydrogen phosphate (monohydrate) | Merck, Germany | |
| Disodium hydrogen phosphate (heptahydrate) | Merck, Germany | 1.06574.1000 |
| Disodium hydrogen phosphate (heptahydrate) | Merck, Germany | |
| Trehalose (dihydrate) | Pfanstiehl | T-104-4 |
| Trehalose dihydrate | | |
| Sucrose | Merck, Germany | 1.07653.9029 |
| Sucrose | Merck, Germany | |
| Methionine | Bafeng Pharmaceuticals & | N/A |
| Methionine | Chemicals, Hubei | |
| Proline | Bafeng Pharmaceuticals & | N/A |
| Proline | Chemicals, Hubei | |
| Glycine | Merck, Germany | 1.00590.1000 |
| Glycine | Merck, Germany | |
| Hydroxypropyl-β-cyclodextrin | Roquette, French | N/A |
| Hydroxypropyl β-cyclodextrin | Roquette, France | |
| Polysorbate 80 | Well, Nanjing | Jiangsu MPA Drug Approval |
| Polysorbate 80 | Well, Nanjing | No. F15423203 |
| Diluted hydrochloric acid Hydrochloric acid | Hunan Er-Kang Pharmaceutical Co., Ltd. | NMPA Drug Approval No. H43020202 |
| | Hunan Er-Kang | |
| Sodium hydroxide | Jinshan, Sichuan | F20100001 |
| Sodium hydroxide | Jinshan, Sichuan | |

| | | |
|---|---|---|
| Note: N/A denotes not available. Trehalose in the following formulae refers to trehalose (dihydrate). | | |

### Preparation and composition of formulations

Buffers of each formula (composition shown in Table 5 except polysorbate 80) were prepared according to the mass concentration in Table using ultrapure water as the solvent, and IEX04-012 protein (prepared according to the method in Example 2 above) was ultrafiltered into each of the buffers. The protein content in each formula solution was adjusted to about 40 mg/mL after the exchange, and then polysorbate 80 was added at a final concentration of 0.3 mg/mL. The solutions were filtered and aliquoted into vials, followed by stoppering and capping. The stability of the above samples was investigated at 40 ± 2 °C.

**Table 5. Information of candidate formulae**

| Serial No. | Formula information |
|---|---|
| Formula 1 F1 | Sodium dihydrogen phosphate (monohydrate) at 0.58 mg/mL (i.e., 4.2 mM), disodium hydrogen phosphate (heptahydrate) at 1.55 mg/mL (i.e., 5.8 mM), trehalose at 80 mg/mL (i.e., 211.5 mM), polysorbate 80 at 0.3 mg/mL, pH 7.0 |
| Formula 2 F2 | Sodium dihydrogen phosphate (monohydrate) at 0.58 mg/mL, disodium hydrogen phosphate (heptahydrate) at 1.55 mg/mL, sucrose at 80 mg/mL (i.e., 233.7 mM), polysorbate 80 at 0.3 mg/mL, pH 7.0 |
| Formula 3 F 3 | Sodium dihydrogen phosphate (monohydrate) at 0.58 mg/mL, disodium hydrogen phosphate (heptahydrate) at 1.55 mg/mL, trehalose at 20 mg/mL (i.e., 52.9 mM), glycine at 15 mg/mL (i.e., 200 mM), polysorbate 80 at 0.3 mg/mL, pH 7.0 |
| Formula 4 F 4 | Sodium dihydrogen phosphate (monohydrate) at 0.58 mg/mL, disodium hydrogen phosphate (heptahydrate) at 1.55 mg/mL, sucrose at 20 mg/mL, glycine at 15 mg/mL, polysorbate 80 at 0.3 mg/mL, pH 7.0 |
| Formula 5 F 5 | Histidine at 1.55 mg/mL (i.e., 10 mM), trehalose at 20 mg/mL, glycine at 15 mg/mL, polysorbate 80 at 0.3 mg/mL, pH 6.0 |
| Formula 6 F 6 | Histidine at 1.55 mg/mL, trehalose at 20 mg/mL, glycine at 15 mg/mL, polysorbate 80 at 0.3 mg/mL, pH 6.5 |
| Formula 7 F7 | Histidine at 1.55 mg/mL, trehalose at 80 mg/mL, polysorbate 80 at 0.3 mg/mL, pH 6.5 |
| Formula 8 F 8 | Sodium dihydrogen phosphate (monohydrate) at 0.58 mg/mL, disodium hydrogen phosphate (heptahydrate) at 1.55 mg/mL, sucrose at 60 mg/mL (i.e., 175.3 mM), proline at 8 mg/mL (i.e., 69.5 mM), polysorbate 80 at 0.3 mg/mL, pH 7.0 |
| Formula 9 F 9 | Sodium dihydrogen phosphate (monohydrate) at 0.58 mg/mL, disodium hydrogen phosphate (heptahydrate) at 1.55 mg/mL, sucrose at 80 mg/mL, hydroxypropyl-β-cyclodextrin at 15 mg/mL (i.e., 10 mM), polysorbate 80 at 0.3 mg/mL, pH 7.0 |
| Formula 10 F 10 | Sodium dihydrogen phosphate (monohydrate) at 0.58 mg/mL, disodium hydrogen phosphate (heptahydrate) at 1.55 mg/mL, sucrose at 80 mg/mL, methionine at 1.5 mg/mL (i.e., 10 mM), polysorbate 80 at 0.3 mg/mL, pH 7.0 |

| | |
|---|---|
| Note: the pH was adjusted with diluted hydrochloric acid or 1M NaOH solution. | |

### Stability study

Detail on experimental conditions and the sampling schedule is shown in Table 6.

**Table 6. Stability investigation scheme**

| Formula | Name of experiment | Experimental scheme and sampling time points |
|---|---|---|
| Formulae 1-10 (i.e., F1-F10) | Forced stability | Preservation at 40 ± 2 °C; sampling at day 0, week 1 and week 2 |

| | | |
|---|---|---|
| Note: after sampling at the above time points, the samples were first cryopreserved in a freezer at -70 °C for later detection, and then thawed before detection as required. | | |

### Criteria

According to the knowledge of the product and the precision of the instrument and the method, criteria for determining the absence of quality change in measurements as compared to the initial values were set, as detailed in Table 1.

**Table 1. Criteria for determining the absence of quality change**

| Test items | Criteria for determining absence of change |
|---|---|
| Appearance (visual inspection) | Clear to slightly opalescent, colorless to pale yellow liquid free of particles |
| Visible particles (test for visible particles) | Conforms to the General Rule 0904 of the Pharmacopoeia of the People's Republic of China (2020 edition, volume III)^{[1]} |
| Protein content (UV method) | Change rate ≤ 10% |
| pH value (method for determining pH) | Change value ≤ 0.3 |
| Purity (SEC-HPLC) | Main peak change ≤ 1.0% |
| Purity (non-reduced CE-SDS) | Main peak change ≤ 2.0% |
| Charge variants (iCIEF) | Changes in main species, acidic species and basic species ≤ 2% |
| Polysorbate 80 content* (HPLC-FLD) | 0.1 mg/ml-0.5mg/ml |
| Biological activity (ELISA) | 70-130% |

| | |
|---|---|
| Note: "*" indicates that the criterion applies only to the formula determination experiment. | |

### Results

The results of the forced stability study are detailed in Table 8. The results show that after standing at 40 ± 2 °C for 1 week, other formulae were qualified in terms of appearance and visible particles than formula 5, which showed heavy opalescence; protein content and pH value demonstrated no significant changes; all formula samples exhibited changes in purity (SEC-HPLC), with the mildest decrease in purity (SEC-HPLC) seen in the sample of formula 7; for charge variants, all formula samples exhibited increased acidic species content and decreased main species, with mildest increase in acidic species content found in the sample of formula 7. After storage at 40 ± 2 °C for 2 weeks, all formula samples exhibited changes in purity (SEC-HPLC), with the mildest decrease in purity (SEC-HPLC) seen in the sample of formula 7; for charge variants, all formula samples exhibited increased acidic species content and decreased main species, with the mildest decrease in main species content found in the sample of formula 7. All formulae showed no change in polysorbate 80 content. All formulae were in the acceptable range of biological activity.

**Table 8. Results of forced stability study**

| Test items | Time | Sample name | | | | | | | | | | |
|---|---|---|---|---|---|---|---|---|---|---|---|---|
| | | Formula 1 F1 | Formula 2 F2 | Formula 3 F3 | Formula 4 F4 | Formula 5 F5 | Formula 6 F6 | Formula 7 F7 | Formula 8 F8 | Formula 9 F9 | Formula 10 F10 | |
| Appearance (Observation) | 0 weeks | Acceptable | Acceptable | Acceptable | Acceptable | Acceptable | Acceptable | Acceptable | Acceptable | Acceptable | Acceptable | |
| | 1 week | Acceptable | Acceptable | Acceptable | Acceptable | Seriously opalescent | Acceptable | Acceptable | Acceptable | Acceptable | Acceptable | |
| | 2 weeks | Acceptable | Acceptable | Acceptable | Acceptable | Turbid | Acceptable | Acceptable | Acceptable | Acceptable | Acceptable | |
| Visible particles (Test for visible particles) | 0 weeks | Acceptable | Acceptable | Acceptable | Acceptable | Acceptable | Acceptable | Acceptable | Acceptable | Acceptable | Acceptable | |
| | 1 week | Acceptable | Acceptable | Acceptable | Acceptable | Seriously opalescent | Acceptable | Acceptable | Acceptable | Acceptable | Acceptable | |
| | 2 weeks | Acceptable | Acceptable | Acceptable | Acceptable | Turbid | Acceptable | Acceptable | Acceptable | Acceptable | Acceptable | |
| Protein content (UV method, mg/mL) | 0 weeks | 38.9 | 42.4 | 39.8 | 40.2 | 40.7 | 39.0 | 38.7 | 38.8 | 39.3 | 39.4 | |
| | 1 week | 39.2 | 42.2 | 39.5 | 39.7 | 39.7 | 38.6 | 38.5 | 39.5 | 39.9 | 39.9 | |
| | 2 weeks | 38.8 | 42.2 | NA | NA | NA | 39.4 | 39.0 | 39.6 | 39.8 | 39.3 | |
| pH value (Method for determining pH) | 0 weeks | 7.2 | 7.2 | 7.1 | 7.2 | 6.1 | 6.3 | 6.4 | 7.1 | 7.1 | 7.0 | |
| | 1 week | 7.2 | 7.2 | 7.1 | 7.1 | 6.0 | 6.3 | 6.4 | 7.1 | 7.1 | 7.1 | |
| | 2 weeks | 7.2 | 7.2 | NA | NA | NA | 6.4 | 6.4 | 7.1 | 7.1 | 7.1 | |
| Purity (SEC-HPLC, %) | Main peak 0 weeks | 99.1 | 99.1 | 99.1 | 99.1 | 99.1 | 99.1 | 99.1 | 99.1 | 99.1 | 99.1 | |
| | Main peak 1 week | 90.6 | 90.4 | 88.1 | 88.0 | 93.9 | 93.1 | 94.1 | 90.2 | 91.4 | 91.2 | |

| Test items | Time | | Sample name | | | | | | | | | |
|---|---|---|---|---|---|---|---|---|---|---|---|---|
| | | | Formula 1 F1 | Formula 2 F2 | Formula 3 F3 | Formula 4 F4 | Formula 5 F5 | Formula 6 F6 | Formula 7 F7 | Formula 8 F8 | Formula 9 F9 | Formula 10 F10 |
| | | 2 weeks | 81.6 | 81.1 | NA | NA | NA | 86.6 | 89.1 | 80.5 | 83.6 | 82.6 |
| | Aggregates | 0 weeks | 0.9 | 0.9 | 0.9 | 0.9 | 0.9 | 0.9 | 0.9 | 0.9 | 0.9 | 0.9 |
| | Aggregates | | | | | | | | | | | |
| | | 1 week | 8.7 | 8.9 | 11.1 | 11.2 | 5.7 | 6.5 | 5.5 | 9.1 | 8.0 | 8.2 |
| | | 2 weeks | 17.1 | 17.6 | NA | NA | NA | 12.6 | 10.2 | 18.2 | 15.3 | 16.1 |
| Purity (non-reduced CE-SDS, %) Purity (nrCE-SDS, %) | Main peak | 0 weeks | 100.0 | 99.9 | 100.0 | 100.0 | 100.0 | 100.0 | 100.0 | 100.0 | 100.0 | 100.0 |
| | Main peak | 1 week | 99.5 | 99.4 | 99.1 | 99.3 | 99.7 | 99.6 | 99.7 | 99.4 | 99.5 | 99.5 |
| | | 2 weeks | 98.8 | 98.9 | NA | NA | NA | 99.3 | 99.3 | 98.8 | 99.0 | 99.0 |
| | Fragments | 0 weeks | 0.0 | 0.1 | 0.0 | 0.0 | 0.0 | 0.0 | 0.0 | 0.0 | 0.0 | 0.0 |
| | Fragments | 1 week | 0.5 | 0.5 | 0.8 | 0.6 | 0.3 | 0.3 | 0.3 | 0.5 | 0.5 | 0.5 |
| | | 2 weeks | 1.1 | 1.0 | NA | NA | NA | 0.6 | 0.6 | 1.1 | 0.9 | 1.0 |
| Charge variants (iCIEF, %) | Acidic species | 0weeks | 38.8 | 38.5 | 39.0 | 38.8 | 39.9 | 39.2 | 39.0 | 39.3 | 39.4 | 39.5 |
| | | 1 week | 43.9 | 43.5 | 45.8 | 46.2 | 43.8 | 43.0 | 43.0 | 44.1 | 43.4 | 43.4 |
| | Acidic species | 2 weeks | 51.0 | 50.8 | NA | NA | NA | 48.5 | 45.5 | 51.5 | 49.9 | 50.3 |
| | Main species | 0weeks | 59.4 | 59.8 | 59.6 | 59.3 | 58.4 | 58.6 | 59.6 | 59.3 | 58.9 | 59.1 |
| | | 1 week | 53.8 | 54.5 | 52.1 | 51.8 | 52.0 | 53.0 | 52.8 | 53.4 | 54.1 | 55.8 |
| | | 2 weeks | 45.4 | 45.6 | NA | NA | NA | 45.5 | 48.3 | 45.2 | 46.6 | 46.1 |

| Test items | Time | | Sample name | | | | | | | | | |
|---|---|---|---|---|---|---|---|---|---|---|---|---|
| | | | Formula 1 F1 | Formula 2 F2 | Formula 3 F3 | Formula 4 F4 | Formula 5 F5 | Formula 6 F6 | Formula 7 F7 | Formula 8 F8 | Formula 9 F9 | Formula 10 F10 |
| | Main species | | | | | | | | | | | |
| | Basic species | 0 weeks | 1.8 | 1.7 | 1.4 | 1.9 | 1.7 | 2.2 | 1.5 | 1.4 | 1.7 | 1.4 |
| | | 1 week | 2.3 | 2.0 | 2.1 | 2.0 | 4.2 | 4.0 | 4.2 | 2.5 | 2.5 | 0.8 |
| | Basic species | 2 weeks | 3.6 | 3.6 | NA | NA | NA | 6.0 | 6.2 | 3.3 | 3.5 | 3.7 |
| Polysorbate 80 content (HPLC-FLD, mg/mL) | 0 weeks | | 0.3 | 0.3 | 0.3 | 0.3 | 0.3 | 0.3 | 0.3 | 0.3 | 0.3 | 0.3 |
| | 1 week | | 0.2 | 0.3 | 0.2 | 0.2 | 0.3 | 0.3 | 0.2 | 0.2 | 0.3 | 0.2 |
| | 2 weeks | | 0.2 | 0.2 | NA | NA | NA | 0.2 | 0.2 | 0.2 | 0.3 | 0.2 |
| Relative binding Activity to Ang2 (ELISA, %) | 0 weeks | | 100 | 100 | NA | NA | 100 | 100 | 100 | 100 | 100 | 100 |
| | 1 week | | 103 | 99 | NA | NA | 82 | 96 | 90 | 92 | 96 | 98 |
| | 2 weeks | | 100 | 102 | NA | NA | NA | 89 | 87 | 91 | 90 | 99 |
| Relative binding Activity to VEGF (ELISA, %) | 0 weeks | | 100 | 100 | NA | NA | 100 | 100 | 100 | 100 | 100 | 100 |
| | 1 week | | 98 | 98 | NA | NA | 98 | 98 | 102 | 101 | 97 | 99 |
| | 2 weeks | | 101 | 99 | NA | NA | NA | 99 | 100 | 102 | 99 | 99 |

| | | | | | | | | | | | | |
|---|---|---|---|---|---|---|---|---|---|---|---|---|
| Note: NA denotes not available. | | | | | | | | | | | | |

Based on the results of the formula determination experiment, formula 7 was determined as the selected formula of IEX04-012. To avoid the addition of hydrochloric acid for pH adjustment in manufacturing, the buffer system was modified to histidine/histidine hydrochloride, and the trehalose concentration was increased to 100 mg/mL (corresponding to an osmolality of 350 mOsmol/kg) due to the low osmolality of formula F7 (255 mOsmol/kg), such that the osmolality of the formulation buffer met the requirements of the Chinese Pharmacopoeia. Hence, the final formula of IEX04-012 was: recombinant anti-vascular endothelial growth factor A (VEGF A)/anti-angiopoietin 2 (Ang2) bispecific antibody at 40.0 mg/mL, histidine at 1.25 mg/mL (8 mM), L-histidine hydrochloride at 0.40 mg/mL (2 mM), trehalose at 100 mg/mL (264.3 mM), and polysorbate 80 at 0.3 mg/mL, pH 6.5. The subsequent long-term stability at 25 °C and 2-8 °C also demonstrated that the IEX04-012 antibody can be stable for a long time under the formula conditions.

The results of the long-term stability test of the final formula 7 are shown in Table 9

**Table 9. Results of formula study**

| Test items | | T0 | 40°C±2°C | | 25°C±2°C | | | 2~8°C | |
|---|---|---|---|---|---|---|---|---|---|
| | | | 2W | 4W | 1M | 2M | 3M | 6M | 12M |
| Appearance (Observation) | | Acceptable | Acceptable | Acceptable | Acceptable | Acceptable | Acceptable | Acceptable | Acceptable |
| Visible particles (Test for visible particles) | | Acceptable | Acceptable | Acceptable | Acceptable | Acceptable | Acceptable | Acceptable | Acceptable |
| Protein content (UV method, mg/mL) | | 40.1 | 40.0 | 40.0 | 39.8 | 39.9 | 40.4 | 40.5 | 40.5 |
| pH value (pH measurement) pH (xx) | | 6.4 | 6.4 | 6.4 | 6.4 | 6.4 | 6.4 | 6.4 | 6.4 |
| Purity (SEC-HPLC, %) | Main peak | 98.9 | 88.9 | 78.0 | 98.4 | 96.9 | 95.5 | 98.8 | 98.5 |
| | Aggregates | 1.1 | 10.2 | 20.6 | 1.6 | 2.3 | 3.4 | 1.2 | 1.5 |
| Purity (non-reduced CE-SDS, %) | Main peak | 99.9 | 98.7 | 97.7 | 99.5 | 99.1 | 98.7 | 99.8 | 99.8 |
| | Fragments | 0.0 | 1.2 | 2.1 | 0.4 | 0.8 | 1.0 | 0.1 | 0.1 |
| Charge variants (iCIEF, %) | Acidic species | 38.4 | 44.6 | 51.6 | 40.3 | 39.0 | 42.5 | 37.9 | 38.7 |
| | Main species | 60.7 | 50.4 | 42.9 | 57.1 | 57.9 | 54.4 | 59.0 | 58.2 |
| | Basic species | 0.9 | 5.0 | 5.5 | 2.6 | 3.1 | 3.1 | 3.0 | 3.1 |
| Polysorbate 80 content (HPLC-FLD, mg/mL) | | 0.3 | N/A | 0.3 | N/A | N/A | N/A | N/A | 0.2 |
| Relative binding Activity to Ang2 (ELISA, %) | | 95 | 86 | 74 | 93 | 84 | 85 | 100 | 96 |
| Relative binding Activity to VEGF A (ELISA, %) | | 103 | 91 | 78 | 98 | 102 | 96 | 99 | 100 |

| | | | | | | | | | |
|---|---|---|---|---|---|---|---|---|---|
| Note: N/A denotes not available. | | | | | | | | | |

### Example 16. High-dose formula study (120 mg/mL) of bispecific antibody of the present invention

In this example, a high-dose (120 mg/mL) bispecific antibody formulation was prepared, and its stability was investigated by monitoring the quality in forcing or accelerating conditions and long-term storage.

A buffer (pH 6.5) containing histidine at 1.25 mg/mL, L-histidine hydrochloride at 0.40 mg/mL, and trehalose at 100.00 mg/mL was prepared. IEX04-012 (prepared according to the method of Example 2) was transferred in the buffer by ultrafiltration, and the protein content was adjusted to about 120 mg/mL; polysorbate 80 was added at a final concentration of 0.3 mg/mL; the solution was filtered and aliquoted into vials, followed by stoppering and capping. The stability of the above samples was examined at 40 ± 2 °C, 25 ± 2 °C, and 2-8 °C, and the specific scheme is shown in Table 10.

**Table 10. Stability investigation scheme**

| Experimenta 1 conditions | Sampling time points | | | Test items |
|---|---|---|---|---|
| 40°C±2°C | 1W | 2W | 4W | Appearance, visible particles, protein content, pH, purity, charge variants, polysorbate 80 content, and biological activity |
| 25°C±2°C | 1M | 2M | 3M | |
| 2~8°C | 3M | 6M | 12M | |

| | | | | |
|---|---|---|---|---|
| Note: after sampling at the above time points, the samples were first cryopreserved at -70 °C for later detection, and then thawed before detection as required. | | | | |

### Criteria

According to the knowledge of the product and the precision of the instrument and the method, criteria for determining the absence of quality change in measurements as compared to the initial values were set, as detailed in Table 1.

| Test items | Criteria for determining absence of change |
|---|---|
| Appearance (visual inspection) | Clear to slightly opalescent, colorless to pale yellow liquid free of particles |
| Visible particles (test for visible particles) | No visible particles |
| Protein content (UV method) | Change rate ≤ 10% |
| pH value (method for determining pH) | Change value ≤ 0.3 |
| Purity (SEC-HPLC) | Monomer change ≤ 1.0% |
| Purity (non-reduced CE-SDS) | Main peak change ≤ 2.0% |
| Charge variants (iCIEF) | Changes in main species, acidic species and basic species ≤ 2.0% |

### Results

The formula study results are detailed in Table 12. The results show that after storage at 40 ± 2 °C for 1 or 2 weeks, the purity assay demonstrated a decrease in the main peak content and an increase in the aggregate content; for charge variants, the main species decreased, while the acidic species increased; results for all other items were acceptable or unchanged. At week 4, significant opalescence was observed. After storage at 25 ± 2 °C for 1 month, 2 months, and 3 months, all samples were qualified for appearance and visible particles. The purity assay (SEC-HPLC) demonstrated a decrease in the main peak content and an increase in the aggregate content; for charge variants, the main species decreased, while the acidic species increased; results for all other items were acceptable or unchanged. After storage at 2-8 °C for 6 months and 12 months, all samples were qualified for appearance and visible particles, while other items showed no significant changes as compared with the initial values.

**Table 12. Results of formula study**

| Test items | | T0 | 40°C±2°C | | | 25°C±2°C | | | 2~8°C | |
|---|---|---|---|---|---|---|---|---|---|---|
| | | | 1W | 2W | 4W | 1M | 2M | 3M | 6M | 12M |
| Appearance (Observation) | | Acceptable | Acceptable | Acceptable | Unacceptable | Acceptable | Acceptable | Acceptable | Acceptable | Acceptable |
| Visible particles (Test for visible particles) | | Acceptable | Acceptable | Acceptable | Significantly opalescent | Acceptable | Acceptable | Acceptable | Acceptable | Acceptable |
| Protein content (UV method, mg/mL) | | 115.5 | 115.5 | 117.7 | | 122.4 | 118.4 | 127.4 | 119.6 | 119.1 |
| pH value (pH measurement) pH (xx) | | 6.2 | 6.2 | 6.2 | | 6.2 | 6.2 | 6.2 | 6.1 | 6.2 |
| Purity (SEC-HPLC, %) | Main peak | 98.7 | 90.9 | 84.0 | | 97.4 | 95.6 | 92.8 | 98.4 | 97.9 |
| | Aggregates | 1.3 | 8.7 | 15.4 | | 2.3 | 3.9 | 6.4 | 1.6 | 1.8 |
| Purity (non-reduced CE-SDS, %) | Main peak | 100.0 | N/A | 98.7 | | 99.5 | 99.0 | 98.1 | 99.4 | 99.6 |
| | Fragments | 0.0 | N/A | 0.9 | N/A | 0.4 | 0.8 | 1.4 | 0.5 | 0.2 |
| Charge variants (iCIEF, %) | Acidic species | 36.5 | 41.6 | 45.3 | | 36.4 | 40.1 | 42.4 | 36.3 | 37.2 |
| | Main species | 60.9 | 53.4 | 48.8 | | 59.3 | 54.6 | 52.2 | 60.0 | 59.6 |
| | Basic species | 2.7 | 5.0 | 5.9 | | 4.4 | 5.3 | 5.3 | 3.7 | 3.2 |
| Polysorbate 80 content (HPLC-FLD, mg/mL) | | 0.5 | N/A | 0.5 | | 0.4 | 0.4 | 0.4 | 0.5 | 0.5 |
| Relative binding Activity to Ang2 (ELISA, %) | | 92 | N/A | 81 | | 93 | 84 | 75 | 96 | 104 |
| Relative binding Activity to VEGF A (ELISA, %) | | 101 | N/A | 113 | | 96 | 92 | 106 | 99 | 104 |

| | | | | | | | | | | |
|---|---|---|---|---|---|---|---|---|---|---|
| Note: N/A denotes not available. | | | | | | | | | | |

As described in Example 15, the buffer system was modified to histidine/histidine hydrochloride to avoid the addition of hydrochloric acid for pH adjustment in manufacturing. The modified IEX04-012 high-dose formula was: recombinant anti-vascular endothelial growth factor A (VEGF A)/anti-angiopoietin 2 (Ang2) bispecific antibody at 120.0 mg/mL, histidine at 1.25 mg/mL, L-histidine hydrochloride at 0.40 mg/mL, trehalose at 100 mg/mL, and polysorbate 80 at 0.3 mg/mL.

### Example 17. High-dose formula study (150 mg/mL) of bispecific antibody of the present invention

In this example, a high-dose (150 mg/mL) bispecific antibody formulation was prepared, and its stability was investigated.

A buffer containing histidine at 1.25 mg/mL, L-histidine hydrochloride at 0.40 mg/mL, and trehalose at 100.00 mg/mL was prepared and adjusted to pH 6.5. IEX04-012 was transferred in the buffer by ultrafiltration, and the protein content was adjusted to about 150 mg/mL; polysorbate 80 was added at a final concentration of 0.3 mg/mL; the solution was filtered and aliquoted into vials, followed by stoppering and capping. The stability of the above samples was examined at 40 ± 2 °C and 25 ± 2 °C.

### Results

The results of the formula study are detailed in Table 13. The results show that after storage at 40 ± 2 °C for 1 or 2 weeks, the purity assay demonstrated a decrease in the main peak content and an increase in the aggregate content; for charge variants, the main species decreased, while the acidic species increased; results for all other items were acceptable or unchanged. After storage at 25 ± 2 °C for 1 and 2 weeks, all samples were qualified for appearance and visible particles. For charge variants, the main species decreased, while the acidic species increased; results for all other items were acceptable or unchanged.

**Table 13. Results of formula study**

| Test items | | T0 | 40°C±2°C | | 25°C±2°C | |
|---|---|---|---|---|---|---|
| | | | 1W | 2W | 1W | 2W |
| Appearance (Observation) | | Acceptable | Acceptable | Acceptable | Acceptable | Acceptable |
| Visible particles (Test for visible particles) | | Acceptable | Acceptable | Acceptable | Acceptable | Acceptable |
| Protein content (UV method, mg/mL) | | 158.7 | 158.7 | 158.7 | 158.7 | 158.7 |
| pH value (Method for determining pH) | | 6.1 | 6.1 | 6.1 | 6.1 | 6.1 |
| Purity (SEC-HPLC, %) | Main peak | 98.7 | 85.9 | 76.4 | 98.4 | 98.0 |
| | Aggregates | 1.3 | 13.8 | 23.1 | 1.6 | 2.0 |
| Purity (non-reduced CE-SDS, %) | Main peak | 99.9 | N/A | 98.4 | N/A | N/A |
| | Fragments | 0.1 | N/A | 0.9 | N/A | N/A |
| Charge variants (iCIEF, %) | Acidic species | 39.0 | 47.3 | 50.9 | 40.4 | 40.2 |
| | Main species | 58.5 | 48.5 | 43.8 | 56.4 | 56.0 |
| | Basic species | 2.5 | 4.2 | 5.3 | 3.2 | 3.9 |
| Polysorbate 80 content (HPLC-FLD, mg/mL) | | 0.3 | N/A | N/A | N/A | N/A |
| Relative binding Activity to Ang2 (ELISA, %) | | 103 | N/A | N/A | N/A | N/A |
| Relative binding Activity to VEGF A (ELISA, %) | | 92 | N/A | N/A | N/A | N/A |

| | | | | | | |
|---|---|---|---|---|---|---|
| Note: N/A denotes not available. | | | | | | |

As described above, the buffer system was modified to histidine/histidine hydrochloride to avoid the addition of hydrochloric acid for pH adjustment in manufacturing. The IEX04-012 high-dose formula was: recombinant anti-vascular endothelial growth factor A (VEGF A)/anti-angiopoietin 2 (Ang2) bispecific antibody at 150.0 mg/mL, histidine at 1.25 mg/mL, L-histidine hydrochloride at 0.40 mg/mL, trehalose at 100 mg/mL, and polysorbate 80 at 0.3 mg/mL.

It should be noted that the formulation of the examples of the present application is applicable to other specific binding molecules of the present application, particularly other bispecific binding molecules in the examples (see sequence listing above), and will achieve comparable results.

The exemplary embodiments of the present invention have been described above. It should be understood by those skilled in the art that these contents are merely exemplary, and various other replacements, adaptations, and modifications can be made within the scope of the present invention. Therefore, the present invention is not limited to the specific embodiments listed herein.

## Claims

1. A liquid formulation, comprising:
(i) a bispecific binding molecule specifically binding to VEGF A and Ang2;
(ii) a buffer;
(iii) a stabilizer, and
(iv) a surfactant,
wherein the pH of the antibody formulation is about 5.0-7.5,
the bispecific binding molecule comprises a first target-binding region specifically binding to VEGF A and a second target-binding region specifically binding to Ang2, wherein the second target-binding region is an anti-Ang2 VHH comprising 3 CDRs: HCDR1, HCDR2, and HCDR3, wherein
the HCDR1 comprises or consists of a sequence set forth in SEQ ID NO: 16;
the HCDR2 comprises or consists of a sequence set forth in SEQ ID NO: 17 or 20; and
the HCDR3 comprises or consists of a sequence set forth in SEQ ID NO: 18;
optionally, wherein the first target-binding region is selected from:
a VHH specifically binding to VEGF A;
an antigen-binding fragment of an antibody, e.g., an scFv, specifically binding to VEGF A, for example, the antibody is a fully human or humanized antibody; or
a VEGF receptor (VEGF R) specifically binding to VEGF A or an extracellular domain thereof or a fusion protein comprising the extracellular domain thereof, e.g., a fusion protein of the extracellular domain thereof with an Fc.

2. The liquid formulation according to claim 1, wherein the anti-Ang2 VHH
(1) comprises or consists of an amino acid sequence set forth in SEQ ID NO: 19 or 21, or comprises an amino acid sequence having at least 90%, 91%, 92%, 93%, 94%, 95%, 96%, 97%, 98%, or 99% identity to the amino acid sequence set forth in SEQ ID NO: 19 or 21; or (2) comprises an amino acid sequence having one or several (preferably no more than 10, 9, 8, 7, 6, 5, 4, 3, 2, or 1) mutations compared to an amino acid sequence set forth in SEQ ID NO: 19 or 21, wherein the mutations are, for example, substitutions, deletions, or additions, preferably substitutions, such as conservative substitutions,
for example, the bispecific binding molecule is a bispecific antibody.

3. The liquid formulation according to claim 1 or 2, wherein the bispecific binding molecule is divalent, trivalent, or tetravalent.

4. The liquid formulation according to any one of claims 1-3, wherein the bispecific binding molecule has the following structure:
light chain variable region VL of the anti-VEGF antibody-linker-heavy chain variable region VH of the anti-VEGF antibody-linker-anti-Ang2 VHH or
heavy chain variable region VH of the anti-VEGF antibody-linker-light chain variable region VL of the anti-VEGF antibody-linker-anti-Ang2 VHH.

5. The liquid formulation according to claim 4, wherein:
the light chain variable region VL of the anti-VEGF antibody comprises LCDR1, LCDR2, and LCDR3, wherein
the LCDR1 comprises or consists of a sequence set forth in SEQ ID NO: 31; the LCDR2 comprises or consists of a sequence set forth in SEQ ID NO: 32; and the LCDR3 comprises or consists of a sequence set forth in SEQ ID NO: 33; and/or
the heavy chain variable region VH of the anti-VEGF antibody comprises HCDR1, HCDR2, and HCDR3, wherein the HCDR1 comprises or consists of a sequence set forth in SEQ ID NO: 35; the HCDR2 comprises or consists of a sequence set forth in SEQ ID NO: 36; and the HCDR3 comprises or consists of a sequence set forth in SEQ ID NO: 37.

6. The liquid formulation according to any one of claims 1-5, wherein
the heavy chain variable region VH of the anti-VEGF antibody comprises or consists of an amino acid sequence set forth in SEQ ID NO: 34, or comprises an amino acid sequence having at least 90%, 91%, 92%, 93%, 94%, 95%, 96%, 97%, 98%, or 99% identity to the amino acid sequence set forth in SEQ ID NO: 34; or the heavy chain variable region VH comprises an amino acid sequence having one or several (preferably no more than 10, 9, 8, 7, 6, 5, 4, 3, 2, or 1) mutations compared to an amino acid sequence set forth in SEQ ID NO: 34, wherein the mutations are, for example, substitutions, deletions, or additions, preferably substitutions, such as conservative substitutions;
the light chain variable region VL of the anti-VEGF antibody comprises or consists of an amino acid sequence set forth in SEQ ID NO: 30, or comprises an amino acid sequence having at least 90%, 91%, 92%, 93%, 94%, 95%, 96%, 97%, 98%, or 99% identity to the amino acid sequence set forth in SEQ ID NO: 30; or the light chain variable region VL comprises an amino acid sequence having one or several (preferably no more than 10, 9, 8, 7, 6, 5, 4, 3, 2, or 1) mutations compared to an amino acid sequence set forth in SEQ ID NO: 30, wherein the mutations are, for example, substitutions, deletions, or additions, preferably substitutions, such as conservative substitutions.

7. The liquid formulation according to any one of claims 4-6, wherein
(1) the bispecific binding molecule comprises or consists of an amino acid sequence set forth in SEQ ID NO: 28, or comprises an amino acid sequence having at least 90%, 91%, 92%, 93%, 94%, 95%, 96%, 97%, 98%, or 99% identity to the amino acid sequence set forth in SEQ ID NO: 28; or
(2) the bispecific binding molecule comprises an amino acid sequence having one or several (preferably no more than 10, 9, 8, 7, 6, 5, 4, 3, 2, or 1) mutations compared to an amino acid sequence set forth in SEQ ID NO: 28, wherein the mutations are, for example, substitutions, deletions, or additions, preferably substitutions, such as conservative substitutions.

8. The liquid formulation according to any one of claims 3-7, wherein the bispecific binding molecule has the following structure:
first anti-VEGF VHH-linker-second anti-VEGF VHH-linker-anti-Ang2 VHH,
wherein the first anti-VEGF VHH is the same as or different from the second anti-VEGF VHH.

9. The liquid formulation according to claim 8, wherein the first anti-VEGF VHH or second anti-VEGF VHH comprises HCDR1, HCDR2, and HCDR3, wherein
the HCDR1 comprises or consists of a sequence set forth in SEQ ID NO: 1; the HCDR2 comprises or consists of a sequence set forth in SEQ ID NO: 2; and the HCDR3 comprises or consists of a sequence set forth in SEQ ID NO: 3;
or
the HCDR1 comprises or consists of a sequence set forth in SEQ ID NO: 6; the HCDR2 comprises or consists of a sequence set forth in SEQ ID NO: 7 or 10; and the HCDR3 comprises or consists of a sequence set forth in SEQ ID NO: 8.

10. The liquid formulation according to claim 8 or 9, wherein the first anti-VEGF VHH or the second anti-VEGF VHH comprises or consists of an amino acid sequence set forth in SEQ ID NO 4, 5, 9, or 11, or comprises an amino acid sequence having at least 90%, 91%, 92%, 93%, 94%, 95%, 96%, 97%, 98%, or 99% identity to the amino acid sequence set forth in SEQ ID NO 4, 5, 9, or 11; or the VHH comprises an amino acid sequence having one or several (preferably no more than 10, 9, 8, 7, 6, 5, 4, 3, 2, or 1) mutations compared to an amino acid sequence set forth in SEQ ID NO: 4, 5, 9, or 11, wherein the mutations are, for example, substitutions, deletions, or additions, preferably substitutions, such as conservative substitutions.

11. The liquid formulation according to any one of claims 8-10, wherein
(1) the bispecific binding molecule comprises or consists of an amino acid sequence set forth in SEQ ID NO: 22, or comprises an amino acid sequence having at least 90%, 91%, 92%, 93%, 94%, 95%, 96%, 97%, 98%, or 99% identity to the amino acid sequence set forth in SEQ ID NO: 22; or
(2) the bispecific binding molecule comprises an amino acid sequence having one or several (preferably no more than 10, 9, 8, 7, 6, 5, 4, 3, 2, or 1) mutations compared to an amino acid sequence set forth in SEQ ID NO: 22, wherein the mutations are, for example, substitutions, deletions, or additions, preferably substitutions, such as conservative substitutions.

12. The liquid formulation according to claim 1, wherein the bispecific binding molecule comprises one or two of the following chains:
VEGF R extracellular domain-Fc-linker-anti-Ang2 VHH.

13. The liquid formulation according to claim 12, wherein the VEGFR extracellular domain is an extracellular domain of VEGFR from a human; preferably, the VEGFR extracellular domain comprises a second antibody-like domain of VEGFR1 and a third antibody-like domain of VEGFR2; more preferably, the VEGFR extracellular domain comprises a second antibody-like domain of human VEGFR1 and a third antibody-like domain of human VEGFR2.

14. The liquid formulation according to claim 12 or 13, wherein the VEGFR extracellular domain comprises or consists of an amino acid sequence set forth in SEQ ID NO: 26, or comprises an amino acid sequence having at least 90%, 91%, 92%, 93%, 94%, 95%, 96%, 97%, 98%, or 99% identity to the amino acid sequence set forth in SEQ ID NO: 26.

15. The liquid formulation according to any one of claims 12-14, wherein the Fc is an Fc derived from human IgG1, IgG2, IgG3, or IgG4, preferably the Fc comprises or consists of an amino acid sequence set forth in SEQ ID NO: 27, or comprises an amino acid sequence having at least 90%, 91%, 92%, 93%, 94%, 95%, 96%, 97%, 98%, or 99% identity to the amino acid sequence set forth in SEQ ID NO: 27.

16. The liquid formulation according to any one of claims 12-15, wherein the VEGF R extracellular domain-Fc is a fusion protein of the VEGFR extracellular domain and the Fc, such as aflibercept or a derivative thereof; for example, the VEGF R extracellular domain-Fc comprises or consists of an amino acid sequence set forth in SEQ ID NO: 25, or comprises an amino acid sequence having at least 90%, 91%, 92%, 93%, 94%, 95%, 96%, 97%, 98%, or 99% identity to the amino acid sequence set forth in SEQ ID NO: 25.

17. The liquid formulation according to any one of claims 12-16, wherein
(1) the bispecific binding molecule comprises or consists of an amino acid sequence set forth in SEQ ID NO: 24, or comprises an amino acid sequence having at least 90%, 91%, 92%, 93%, 94%, 95%, 96%, 97%, 98%, or 99% identity to the amino acid sequence set forth in SEQ ID NO: 24; or
(2) the bispecific binding molecule comprises an amino acid sequence having one or several (preferably no more than 10, 9, 8, 7, 6, 5, 4, 3, 2, or 1) mutations compared to an amino acid sequence set forth in SEQ ID NO: 24, wherein the mutations are, for example, substitutions, deletions, or additions, preferably substitutions, such as conservative substitutions.

18. The liquid formulation according to any one of claims 1-17, wherein the linker comprises or consists of an amino acid sequence set forth in SEQ ID NO: 23.

19. The liquid formulation according to any one of claims 1-18, wherein the buffer is selected from one or more of histidine, histidine salt, glutamate, phosphate, acetate, citrate, and tris(hydroxymethyl)aminomethane; preferably, the buffer is selected from a combination of histidine and histidine hydrochloride, phosphate, and histidine; preferably, the phosphate is a combination of disodium hydrogen phosphate and sodium dihydrogen phosphate.

20. The liquid formulation according to any one of claims 1-19, wherein the stabilizer is selected from one or more of a saccharide, a polyol, and an amino acid or a salt thereof,
preferably the saccharide is selected from: sucrose, dextrose, lactose, maltose, trehalose, cyclodextrin, maltodextrin, and glucan; the polyol is selected from: mannitol, sorbitol, and xylitol; the amino acid or the salt thereof is selected from: arginine, glycine, proline, methionine, an arginine salt, and a combination thereof; the cyclodextrin is preferably hydroxypropyl-β-cyclodextrin,
more preferably, the stabilizer is sucrose or trehalose, or a combination of sucrose or trehalose with an additional component selected from one or more of the following: glycine, proline, methionine, and hydroxypropyl-β-cyclodextrin.

21. The liquid formulation according to any one of claims 1-20, wherein the surfactant is selected from polysorbate-20, polysorbate-80, polysorbate-60, polysorbate-40, or pluronic, preferably polysorbate-80.

22. The liquid formulation according to any one of claims 1-21, wherein the concentration of the antibody or antigen-binding fragment thereof is about 1-200 mg/mL, preferably 30-150 mg/mL; and/or
the concentration of the buffer is about 0.5-200 mM, preferably 5-30 mM; and/or
the concentration of the stabilizer is about 1-1000 mM, preferably about 50-500 mM; and/or
the concentration of the surfactant is about 0.01-10 mg/mL, preferably 0.05-2 mg/mL; and/or
the pH of the liquid formulation is about 6.1-7.0, preferably about 6.3-6.8.

23. The liquid formulation according to any one of claims 1-22, wherein the liquid formulation is an injection or an eye drop, more preferably an intravitreal injection.

24. The liquid formulation according to any one of claims 1-18, comprising:
(i) the bispecific binding molecule at about 10-160 mg/mL;
(ii) the buffer at about 5-20 mM, the buffer being histidine or a combination of histidine and histidine hydrochloride;
(iii) trehalose at about 200-350 mM; and
(iv) polysorbate 80 at about 0.1-1 mg/mL; wherein
a pH of the liquid formulation is about 6.1-7.0, preferably about 6.5.

25. The liquid formulation according to claim 24, comprising:
(i) the bispecific binding molecule at about 30-150 mg/mL;
(ii) histidine at about 8-12 mM;
(iii) trehalose at about 200-300 mM; and
(iv) polysorbate 80 at about 0.2-0.4 mg/mL; wherein
the pH of the liquid formulation is about 6.1-6.6, preferably about 6.5.

26. The liquid formulation according to claim 24, comprising:
(i) the bispecific binding molecule at about 30-150 mg/mL;
(ii) histidine at about 1-3 mM and histidine hydrochloride at about 7-9 mM;
(iii) trehalose at about 250-280 mM; and
(iv) polysorbate 80 at about 0.2-0.4 mg/mL; wherein
the pH of the liquid formulation is about 6.1-6.6, preferably about 6.5.

27. A solid formulation obtained by solidifying the liquid formulation according to any one of claims 1-26, wherein the solid formulation is, e.g., in the form of a lyophilized powder for injection.

28. A delivery device, comprising the liquid formulation according to any one of claims 1-26 or the solid formulation according to claim 27, e.g., in the form of a pre-filled syringe.

29. Use of the liquid formulation according to any one of claims 1-26 or the solid formulation according to claim 27 in preparing a medicament or delivery device for preventing or treating an ocular disease associated with angiogenesis, such as an ocular disease associated with corneal neovascularization.
